# EUROPEAN PATENT APPLICATION

(11) **EP 1 586 657 A1**
(43) Date of publication of application: **19.10.2005**
(21) Application number: 05006769.3
(22) Date of filing: 29.03.2005
(51) Int. Cl.: C12Q 1/61

(54) **Method of diagnosing depression**

(30) Priority: 29.03.2004 JP 2004096068; 18.02.2005 JP 2005042534
(71) Applicant: Hitachi Ltd., Tokyo 100-8280 (JP); Rokutan, Kazuhito, Osaka-shi Osaka 532-0002 (JP); Ohmori, Tetsuro, Tokushima-shi Tokushima 770-8041 (JP)
(72) Inventor: Rokutan, Kazuhito Dept. of Stress Science, 3-18-15, Kuramoto-cho Tokushima 770-8053 (JP); Ohmori, Tetsuo Dept. of Psychiatry, 3-18-15, Kuramoto-cho Tokushima 770-8053 (JP); Morita, Kyoko Dept. of Stress Science, 3-18-15, Kuramoto-cho Tokushima 770-8053 (JP); Ohta, Masayuki c/o Hitachi, Ltd., Chiyoda-ku Tokyo 100-8220 (JP); Saito, Toshiro c/o Hitachi, Ltd., Chiyoda-ku Tokyo 100-8220 (JP)
(74) Representative: Strehl Schübel-Hopf & Partner

(57) **Abstract**

This invention provides a novel method of diagnosing the conditions of depression of a patient in a simple, objective, and accurate manner In this method, gene expression is analyzed using mRNA of a subject's peripheral blood to evaluate whether or not the subject is afflicted with depression, the type of depression of a subject who had been evaluated as being afflicted with depression is identified, and the conditions of depression are then diagnosed in accordance with the type of depression.

## Description

The present application claims priority from Japanese applications JP 2004-096068 filed on March 29, 2004 and JP 2005-042534 filed on February 18, 2005, the contents of which are hereby incorporated by reference into this application.

### Technical Field

The present invention relates to a method of diagnosing depression. More particularly, the present invention relates to a method of diagnosing depression, wherein gene expression is analyzed using mRNA of patients' peripheral bloods to cluster patients afflicted with depression, and conditions thereof are then diagnosed.

### Background Art

Depression is a disease with high lifetime morbidity of approximately up to 10%, and this rate is predicted to further increase in the future due to stress in contemporary society. This disease seriously afflicts patients mentally and physically and imposes enormous damage upon their social lives. In addition, it is a serious disease that often leads to suicide. It is deduced that many of the people who commit suicide (as many as 30,000 or more per year in Japan) are afflicted with depression. This disease is also deeply associated with societal problems such as truancy, unemployment, and social withdrawal or medical problems such as alcohol-related disorders. Establishment of methods of precisely diagnosing and promptly treating this disease is indispensable for improving the quality of life, and thus is an urgent need of society as a whole.

Diagnosis of depression is, however, far from simple. Cardinal symptoms of depression are, for example, depressive mood, hypobulia, loss of interest and pleasure, disrupted concentration and attention, lowered self-esteem and self-confidence, feelings of guilt and worthlessness, pessimism about the future, thoughts of suicide, sleep disorders, and loss of appetite. These symptoms have features peculiar to depression, which differ from depressed feelings experienced by anyone, and also differ from the lowered mental activity and sense of exhaustion experienced by people afflicted with physical diseases. The symptoms of depression are mainly comprehended by taking a precise medical history, questioning when and how the symptoms in terms of mental activity were developed and what types of damages have been imposed upon their social and domestic lives, and confirming various symptoms based on a patient's attitude or the contents of conversations during consultation. For example, family medical history, anamnesis, physical conditions, early developmental history, life history, personality inclination, premorbid social adaptation, and the occurrence of any episode(s) that had triggered the disease can be important references. In order to accurately comprehend these factors, an interview needs to be conducted by a highly skilled specialist in psychiatric medicine for approximately 1 hour. Further, it should be confirmed that a patient does not have any major abnormalities in terms of general physical or neurological conditions. If necessary, the possibility of the existence of organic brain disorders is to be eliminated by electroencephalography or brain imaging tests. The patient is then subjected to diagnosis. The findings are compared with the diagnostic standards issued by the World Health Organization (WHO) or the American Psychiatric Association, and the diagnosis can be generally confirmed.

As a major drawback, conventional diagnostic methods require skilled techniques. Needless to say, thorough knowledge and practice concerning depression are required. However, there are numerous psychological, mental, and physical states that result in the exhibition of depressive conditions even though they are not forms of depression. Differential diagnosis also becomes essential. Accordingly, diagnosis must be conducted by a thoroughly trained specialist in psychiatric medicine. Depression, which is a common disease with lifetime morbidity of approximately 10%, however, is often the subject of consultation with primary care doctors. Diagnosis of depression without objective medical findings is not always easy for general doctors who may not be acquainted with psychiatric consultation. Depression is a medical disease that requires treatment of the body (brain), including medication. Accordingly, it is difficult for specialists in clinical psychology, such as clinical psychotherapists, or mental health workers, such as public health nurses, to independently diagnose depression.

Technical skill is required for diagnosis mainly because of a lack of simple and objective methods of diagnosis regarding symptoms. Although there is a screening method utilizing a self-administered questionnaire, people tend to fill in the questionnaire based on their subjective viewpoints. Thus, genuine depression cannot be distinguished from depressed feelings caused by personality-based factors, environmental factors, or poor physical conditions. Symptom rating scales employed by doctors are often used in determination of severity, although adequate questioning is required to evaluate each item. Thus, such methods cannot be alternatives to diagnosis.

Many testing methods have been heretofore attempted, with the aim of utilizing them as objective indicators. Depression causes functional alteration in brain monoamine systems. This alteration is known to have a considerable influence upon the neuroendocrine system, the neuroimmune system, and the autonomic nervous system via psychosomatic correlation. In particular, the application of the results of a dexamethasone suppression test that allows accurate comprehension of neuroendocrine abnormalities, i.e., a minor level of adrenal cortical hormone hypersecretion, to diagnosis of depression has been extensively examined from the 1980s onwards. Clinical application thereof was, however, not realized due to the necessity for complicated procedures such as the administration of test drugs and limitations in terms of sensitivity or specificity. At the study phase, other abnormalities in the neuroendocrine system, the neuroimmune system, the autonomic nervous system, circadian rhythms, sleep architecture, and the like had been reported. Recently, changes regarding conditions of brain blood flow or brain monoamine receptors are also pointed out as objective indicators, although they are still disadvantageous in terms of sensitivity and reproducibility. Given the aforementioned factors, diagnosis of a complicated psychiatric disease, i.e., depression, is difficult by a method of testing limited factors. Enormous amounts of time and labor are required to perform conventional testing methods and to diagnose the disease. From the viewpoint of simplicity, conventional techniques cannot be applied to routine medical care at present.

In the past, the catecholamine hypothesis, the indoleamine hypothesis, the GABA hypothesis, the glutamine hypothesis, the dopamine hypothesis, the neurogenesis hypothesis, and the like have been proposed as causes of depression. Many discrepancies of these hypotheses have been pointed out, and they have not yet resulted in conclusions. Linkage studies and association studies based on molecular genetic engineering and the search for sensitive domains of chromosomes by linkage analysis have been carried out. In the case of a disease such as depression, the diathesis (biological feature) of which is generated through interactions among multiple genes and environmental factors such as stress, therefore analysis of the pathogenic gene is extremely difficult. Based on past gene analysis, genes such as those related to serotonin transporter, serotonin 1A/2C receptor, dopamine D2/D3 receptor, dopamine transporter, tyrosine hydroxylase, tryptophan hydroxylase, monoamine oxidase, and ATPase have been reported as candidate functional genes associated with depression. For example, the correlation between Na/K-ATPase and psychiatric diseases, such as depression (Depress Anxiety 1997, 5, pp. 53-65) or dysthymia (J. Basic Clin. Physiol. Pharmacol. 2000, 11 (4), pp. 375-94), has been pointed out. Improvement of symptoms caused by an antidepressant, i.e., carbamazepine, is reported to be correlated with elevation of erythrocyte Na/K-ATPase activity (Neuropsychobiology 1999, 40 (3), pp. 134-9). Some researchers are, however, skeptical about the aforementioned reports, and additional tests have been conducted thereon.

### Summary of the Invention

An object of the present invention is to provide a novel method of diagnosing the conditions of depression of a subject in a simple, objective, and accurate manner.

The present inventors have focused on peripheral leukocytes that can be easily obtained as specimens and allow many receptors of factors associated with stress responses to be expressed therein in order to objectively diagnose the conditions of depression, in the development of which stress plays an important role. They have extensively analyzed the expression patterns of mRNAs of approximately 1,500 genes associated with stress responses and then developed certain patterns. Thus, they have found a method that is capable of classification patients afflicted with depression and diagnosing the conditions thereof. This has led to the completion of the present invention.

More specifically, the present invention relates to a method of diagnosing depression, wherein gene expression is analyzed using mRNA of a subject's peripheral blood to evaluate whether or not the subject is afflicted with depression, the type of depression of a subject who had been evaluated as being afflicted with depression is identified, and the conditions of depression are then diagnosed in accordance with the type of depression.

According to this method, the expression profiles of the marker gene for depression (an indicator for evaluating whether or not a subject has been afflicted with depression) selected from among the genes listed in Table 1 can be employed to evaluate whether or not a subject is afflicted with depression. When a subject was evaluated as being afflicted with depression, the expression profiles of the marker gene for classification (an indicator for classifying a patient afflicted with depression) selected from among the genes listed in Table 2 can be employed to identify the type of depression in the subject to be type PA or PB.

ATP2A2, SCYA5, STIP1, EEF1A1, GRB10, CASP6, TSSC1, RAB9, NFATC3, and TPR are particularly useful marker genes for depression. GNG10, CLK1, P2Y5, IFNGR1, TAF2F, PIM1, MAP2K3, HDGF, INSR, and COX6C are particularly useful marker genes for classification.

When a subject was evaluated to have type PA depression, the expression profile of the marker gene for diagnosing type PA depression (an indicator for the conditions or a course of treatment of a patient with type PA depression) selected from among the genes listed in Table 3 can be employed to more precisely diagnose the conditions thereof. When a subject was evaluated to have type PB depression, the expression profile of the marker gene for diagnosing type PB depression (an indicator for the conditions or a course of treatment of a patient with type PB depression) selected from among the genes listed in Table 4 can be employed to more precisely diagnose the conditions thereof.

CDC10, GZMA, TNFRSF6, HSPCA, NR3C1, TOPBP1, ARNTL, RAP1A, POLR2B, and ITGB1 are particularly useful marker genes for depression. POU2F2, BCL2L1, DAXX, COX4, CD3G, FCER1G, NME2, CPT1B, HSPE1, and COX7A2 are particularly useful marker genes for classification.

According to another embodiment of the present invention, the expression profiles of the marker gene for depression selected from among the genes listed in Table 7 can be employed to evaluate whether or not a subject is afflicted with depression. When a subject was evaluated to be afflicted with depression, the expression profiles of the marker gene for classification selected from among the genes listed in Table 8 can be employed to identify the type of depression to be type PA or PB.

HLA-G, HRH4, PSMB9, ATP2A2, SCYA5, SLC6A4, CASP6, CSF2, HSD3B1, and RAB9 are particularly useful marker genes for depression. HSPE1, PSMA4, ADH5, PSMA6, COX17, HMG1, GPR24, COX6C, FGF2, and COX7C are particularly useful marker genes for classification.

When a subject was evaluated to have type PA depression, the expression profile of the marker gene for diagnosing type PA depression selected from among the genes listed in Table 9 can be employed to more precisely diagnose the conditions thereof. When a subject was evaluated to have type PB depression, the expression profile of the marker gene for diagnosing type PB depression selected from among the genes listed in Table 10 can be employed to more precisely diagnose the conditions thereof.

CLK1, PSMC6, TAF2F, P2Y5, CASP3, HSPCA, MSH2, SLC38A2, B2M, and AKAP11 are particularly useful marker genes for diagnosing type PA depression. CCNA2, HGF, GPR24, PTGER3, COX7A2, BDKRB2, UFD1L, HMG1, PSMA4, and ATP6J are particularly useful marker genes for diagnosing type PB depression.

According to the method of diagnosing depression of the present invention, the course of treating a single subject who had been diagnosed to be afflicted with depression can be accurately evaluated by comparing and analyzing the gene expression profiles before and after the treatment of the subject.

The methods of analyzing gene expression that are employed in the present invention are not particularly limited. DNA-immobilized solid substrates, such as chips, arrays, membrane filters, and capillaries, are preferable.

The present invention also provides a solid substrate for diagnosing depression having immobilized thereon probes that each independently specifically hybridize to any one of the genes listed in Tables 1 to 4 for detecting the target gene. Preferably, the target genes at least include ATP2A2, SCYA5, STIP1, EEF1A1, GRB10, CASP6, TSSC1, RAB9, NFATC3, and TPR listed in Table 1, GNG10, CLK1, P2Y5, IFNGR1, TAF2F, PIM1, MAP2K3, HDGF, INSR, and COX6C listed in Table 2, CDC10, GZMA, TNFRSF6, HSPCA, NR3C1, TOPBP1, ARNTL, RAP1A, POLR2B, and ITGB1 listed in Table 3, and POU2F2, BCL2L1, DAXX, COX4, CD3G, FCER1G, NME2, CPT1B, HSPE1, and COX7A2 listed in Table 4.

According to another embodiment of the present invention, the present invention provides a solid substrate for diagnosing depression having immobilized thereon probes that each independently specifically hybridize to any one of the genes listed in Tables 7 to 10 for detecting the target gene. Preferably, the target genes at least include HLA-G, HRH4, PSMB9, ATP2A2, SCYA5, SLC6A4, CASP6, CSF2, HSD3B1, and RAB9 listed in Table 7, HSPE1, PSMA4, ADH5, PSMA6, COX17, HMG1, GPR24, COX6C, FGF2, and COX7C listed in Table 8, CLK1, PSMC6, TAF2F, P2Y5, CASP3, HSPCA, MSH2, SLC38A2, B2M, and AKAP11 listed in Table 9, and CCNA2, HGF, GPR24, PTGER3, COX7A2, BDKRB2, UFD1L, HMG1, PSMA4, and ATP6J listed in Table 10.

The present invention further provides a system for diagnosing depression for performing the method of diagnosing depression of the present invention. This system comprises a means for comparing and analyzing the gene expression data of a subject with that of a healthy volunteer and of a patient afflicted with depression, which had been previously obtained, and can diagnose the conditions of depression of the subject in accordance with the type of depression.

Preferably, the aforementioned system further comprises a means of comparing and analyzing the gene expression data of a subject, of a healthy volunteer, and of a patient afflicted with depression in combination with the data concerning their age and sex.

In the present invention, gene expression is analyzed using patients' peripheral bloods to cluster patients afflicted with depression, and conditions thereof or the course of treatment are then diagnosed. Thus, depression can be diagnosed in a non-invasive, simple, and accurate manner.

### Brief Description of the Drawings

Fig. 1 shows the groups of genes exhibiting significant differences between patients and healthy volunteers. Shading indicates the difference in expression levels of 10 or lower.
Fig. 2 shows the groups of genes exhibiting significant differences between the PA group and the PB group. Shading indicates the difference in expression levels of 10 or lower.
Fig. 3 shows the groups of genes exhibiting significant differences before/after treatment in the PA group. Shading indicates the difference in expression levels of 10 or lower.
Fig. 4 shows the groups of genes exhibiting significant differences before/after treatment in the PB group. Shading indicates the difference in expression levels of 10 or lower.
Fig. 5 schematically shows the method of diagnosing depression according to the present invention; wherein F1 indicates a DNA chip, F2 indicates probe DNA corresponding to the gene selected in the present invention, F3 indicates an excitation light source and a fluorescence detector, and F4 indicates a computer for regulating a fluorescence detector.
Fig. 6 schematically shows the system of diagnosing depression according to the present invention; wherein a database of personal information stores information such as sex and age.
Fig. 7 shows clustering of patient/healthy volunteer comparison.
Fig. 8 shows the gene expression data of subjects of the PA group. Shading indicates the difference in expression levels of 10 or lower.
Fig. 9 shows the gene expression data of subjects of the PB group. Shading indicates the difference in expression levels of 10 or lower.
Fig. 10 is a colored chart showing the results of cluster analysis for the group of genes with varying expression levels common in the patient group.
Fig. 11 is a colored chart showing the results of cluster analysis for the patients/healthy volunteers.
Fig. 12 is a colored chart showing the results of cluster analysis between a patient and a healthy volunteer and before/after treatment in the PA group.
Fig. 13 is a colored chart showing the results of cluster analysis between a patient and a healthy volunteer and before/after treatment in the PB group.
Fig. 14 is a colored chart showing the results of cluster analysis for the group of genes with varying expression levels common in the patient group.
Fig. 15 is a colored chart showing the results of cluster analysis for the patients/healthy volunteers.
Fig. 16 is a colored chart showing the results of cluster analysis between a patient and a healthy volunteer (P) and before/after treatment (N) in the PA group.
Fig. 17 is a colored chart showing the results of cluster analysis between a patient and a healthy volunteer (P) and before/after treatment (N) in the PB group.

### Detailed Description of the Invention

### 1. Marker genes for diagnosing depression

The present inventors extracted RNA from the whole blood collected from patients and healthy volunteers as described below, and gene expression of patients was then analyzed using DNA chips, along with that of healthy volunteers. The marker genes were determined based on the results. A DNA chip comprises DNA fragments having nucleotide sequences corresponding to numerous genes immobilized on a substrate such as a glass substrate, and it is used for detecting RNA in a sample by hybridization. Instead of the aforementioned DNA chip, other DNA-immobilized solid substrates (such as DNA arrays, capillaries, or membrane filters) or quantitative assay techniques may be employed, as long as extensive analysis of gene expression is feasible.

Target patients were those who had agreed with the written description for participating in the research for developing the present diagnostic method selected from among untreated patients afflicted with depression. Patients with serious physical complications or those taking therapeutic agents for physical diseases were excluded. Diagnosis was made in accordance with a depressive episode specified in the International Classification of Diseases, 10th revision (ICD-10). Healthy volunteers with the same sex and age conditions were selected for each of the patients for comparison.

Differences in gene expression levels between samples obtained from patients and samples obtained from healthy volunteers or those between samples obtained from a single patient before and after treatment were determined. A group of genes having fluorescence intensities of 300 or higher in both of the data on patient/healthy volunteer comparison and the data on before/after treatment comparison was selected as the target genes.

Among the data on patient/healthy volunteer comparison, the gene with a significantly higher or lower expression level was selected via a significant difference test. The gene of the patient with significantly higher or lower expression level compared to that of the healthy volunteer was then selected as an indicator for evaluating whether or not the patient has been afflicted with depression, i.e., as the "marker gene for depression."

Subsequently, the data on patient/healthy volunteer comparison was subjected to cluster analysis employing all the target genes (hierarchical clustering based on the cosine coefficient distance without a weight between clusters). As a result, the present inventors found that the patient/healthy volunteer comparison samples were roughly divided into two groups, i.e., the PA group and the PB group. The tests were carried out between groups, and the gene that was peculiar to each group was selected as an indicator for classifying a patient afflicted with depression, i.e., as the "marker gene for classification" of the patient afflicted with depression.

Based on the above results, the data on before/after treatment comparison was grouped. The data on patient/healthy volunteer comparison and the data on before/after treatment comparison were aligned for each patient in each group, and the data were compared and analyzed. The group of genes with reversed expression patterns between the data on patient/healthy volunteer comparison and the data on before/after treatment comparison was extracted. The reversed expression patterns between the data on patient/healthy volunteer comparison and the data on before/after treatment comparison indicate a change in gene expression that is observed characteristically when the patient afflicted with depression received treatment involving the use of an antidepressant. Specifically, the extracted group of genes is useful as an indicator for the conditions or the course of treatment of the patients afflicted with depression in each group. This group of genes was selected as the "marker genes for diagnosing each group (e.g., the marker genes for diagnosing type PA depression and the marker genes for diagnosing type PB depression)."

Expression levels of the marker gene was employed as an indicator to evaluate whether or not the subject had been afflicted with depression and the course of treatment by classification. This result was very consistent with the results of clinical finding. Thus, the marker genes according to the present invention were found to be effective.

### 2. Association between marker gene and depression

At present, mechanisms of depression are indefinite, although the following is known as a correlation between the group of genes selected as marker genes and depression or other psychiatric diseases.

The genes, the expression levels of which had been significantly varied in the patient/healthy volunteer comparison samples, contained a large number of cytokine-associated genes, such as SCYA5 encoding a T-cell-specific protein, TNFRSF9 or TNFSF10 belonging to the TNF superfamily, or ILIR2 or IL2RB (an interleukin receptor). The association between cytokine and depression has been pointed out. Inflammatory cytokines such as interleukins (IL)-1, 6, and 8 are associated with stress responses, and affect the central nervous system, thereby causing drowsiness, loss of appetite, and other symptoms. As a major side effect of interferon α used for treating hepatitis C, development of depression is well known. Based on the results attained via the present invention, significant changes in the expression level of cytokine-associated genes were observed in patients afflicted with depression, in the development of which stress may be involved, as anticipated. In particular, the expression level of interferon-associated genes was significantly changed. Thus, development of depression is considered to be associated with interferon therapy. Therefore, analysis of mRNA expression patterns of factors regulating functions of immune system cells was considered to be very useful for diagnosing depression.

It has been pointed out that ATRX is associated with X-chromosome-linked mental retardation (e.g., ATR-X syndrome, Carpenter syndrome, Juberg-Marsidi syndrome, or Smith-Fineman-Myers syndrome).

The expression level of the genes associated with the renin-angiotensin system, such as NR3C1 and SGK2, was found to vary in the case of patients afflicted with depression before and after treatment. Association of the renin-angiotensin system and sporadic Alzheimer's disease has been pointed out (Eur J Hum Genet. 2001: 9(6): 437-444). Also, association of the angiotensin-converting enzyme (ACE) gene polymorphism with schizophrenia has also been analyzed (Neuropsychobiology 2001; 44(1): 31-35).

Recently, the concept of perceiving clinical conditions involved with ion channel dysfunctions as "channel diseases" has been proposed. An ion channel serves as the most important function for neuron cell activity, and its association with epilepsy, ataxia, migraine, schizophrenia, Alzheimer's disease, and other neurodegenerative diseases has been pointed out (CNS Drug Rev 2001; 7(2): 214-240). Concerning Na/K-ATPase and psychiatric diseases, association of the ion channel with depression (Depress Anxiety 1997, 5, pp. 53-65) or dysthymia (J. Basic Clin. Physiol. Pharmacol. 2000, 11 (4), pp. 375-94) has been particularly noted. For example, the association between the Na/K-ATPase α subunit ATP1A3 (Biol Psychiatry 1998; 44: 47-51) or β subunit ATP1B3 (Biol Psychiatry 1995; 37: 235-244) and bipolar disorders has been reported. Further, improvement of symptoms caused by an antidepressant, carbamazepine, is known to be correlated with elevation of erythrocyte Na/K-ATPase activity (Neuropsychobiology 1999, 40 (3), pp. 134-9). ATP1B3P1 is a pseudogene of ATP1B3 and is transcribed from the same genome. In the present invention, changes in the mRNA expression patterns of the gene encoding ATPase, such as ATP2A2, ATP2C1, ATP5JD, or ATP6H, reflect the state of depression. Accordingly, it was suggested that these genes were associated with depression in one way or another.

The expression level of the heat shock protein (HSP) family that is induced by a variety of forms of environmental stress and that contributes to the acquisition of stress responsiveness and stress resistance of cells also showed relatively major variation in leukocytes of patients afflicted with depression. mRNA expression levels were varied in HSPCB, HSPD1, HSPA10, or HSPA4. These HSP families are considered to be a group of genes important for the diagnosis of depression.

At present, mRNA expression levels of RNA polymerase II subunits or binding protein genes were both found to have been lowered, and their expression levels were found to have been restored as the disease state reached a state of remission, although association thereof with depression has not yet been clarified. Expression levels of a group of polymerase-associated genes, such as 140 kDa RNA polymerase II subunit protein gene (POLR2B), RNA polymerase II transcription elongation factor B (SIII) polypeptide 1 (TCEB1), RNA polymerase II transcription elongation factor B (SIII) polypeptide 1 homolog (TCEB1L), poly(A) polymerase, RNA polymerase β subunit, RNA polymerase III, and UDP-galactose transporter novel isozyme (SLC35AI), reflected conditions of depression.

Recently, research into the causes of depression in relation to receptor signalings and transcription factors mediating distinct gene expressions has drawn attention, in addition to the search for association of metabolism of neurotransmitters including monoamine or receptors themselves with depression. A monoamine receptor is a 7-transmembrane G-protein-coupled receptor that activates inositol phosphate cycles and protein kinase C (PKC). This receptor also activates the elevation of cyclic AMP and the protein kinase A (PKA) pathway. Further, transcription factors activated by these signal transducing molecules and their gene products are focused, and it is expected that associations of these pathways with functional disorders will be discovered. Lithium derivatives, the effects of which as mood stabilizers for patients afflicted with bipolar disorders have been verified, are actually reported to act on signal-transducing pathways such as G-proteins, inositol phosphate cycles, PKC, PKA, glycogen synthase kinase 3-β, or Akt cascade, thereby exhibiting pharmacological actions (Br J Psychiatry 2001; 41: suppl 128-133).

Evidence that would support such reports was found in a group of genes associated with conditions of depression. Lowered mRNA expression levels of signal-transducing factors, such as PKCη (PRKCH), PKCβ1 isozyme, and phosphoinosidite 3'-kinase α subunit (PIK3CA), were observed. Lithium inactivates glycogen synthase kinase 3 and intensifies Wnt signals. In the case of patients afflicted with depression, expression levels of connective tissue growth factor-associated protein WISP-3, β-catenin (CTNNB1), and transcription factor E2A (TCF3) were lowered, and their expression levels were restored as the symptoms reached a state of remission. Lowered mRNA expression levels of GTP-binding proteins, i.e., RAB4 and RAB7L1, were observed, and their restoration through treatment was observed.

Concerning growth factor-associated proteins, mRNA expression levels of TGF-β receptor, TGF-β-induced clone 22 homolog (TSC22), and the insulin signal transducing molecule IRS4, reflected the symptoms of depression. In addition, mRNA expression levels of anti-oncogenes, i.e., Rb-associated protein RBBP7 and growth inhibitory factors ING1 and PTEN, were all lowered in patients afflicted with depression, and these expression levels were restored as the disease condition reached a state of remission. In a reflection of the expression patterns of these growth-associated genes, mRNA expression levels of CDKN2C, CDK7, CCNB2, and CCNG1 associated with a cell cycle were all lowered, and lowered mRNA expression levels of topoisomerase IIβ and topoisomerase II-binding protein (TOPBP1) associated with DNA replication were observed. The evidence that suggests lowered general mitogen activity was observed in leukocytes of patients afflicted with depression. Expression levels of these genes were also restored as the symptoms reached a state of remission. Lowered mRNA expression levels of the DNA repair enzyme MSH6, an apoptosis signal molecule DAP3 or API1, and caspase 10 were associated with symptoms of patients afflicted with depression. When variations in growth-associated genes were examined altogether, a cell cycle was deduced to be generally lowered in leukocytes of patients afflicted with depression.

### 3. Method for diagnosing depression and system for diagnosing depression

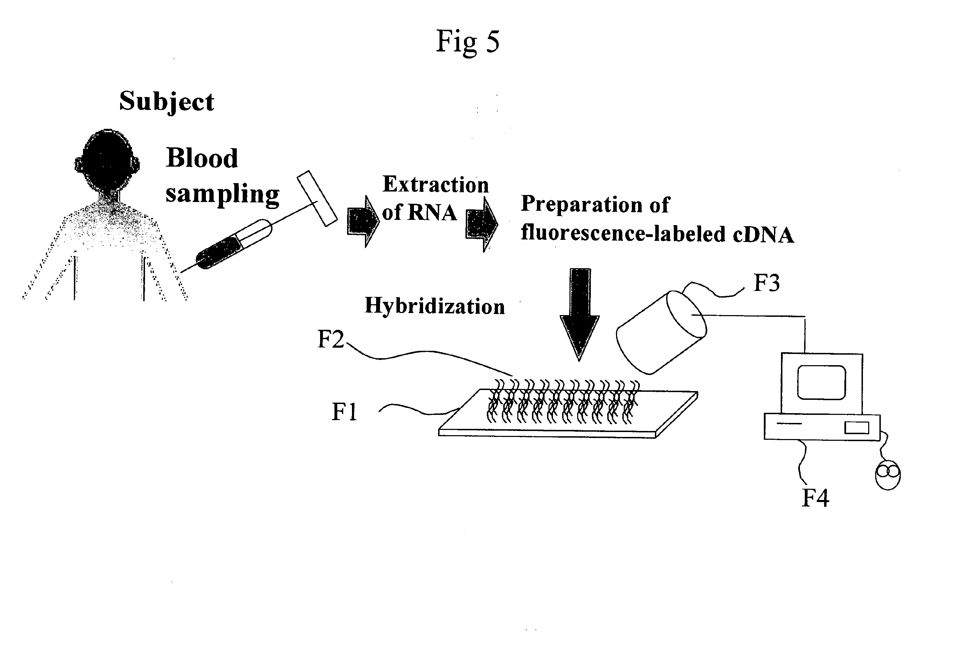

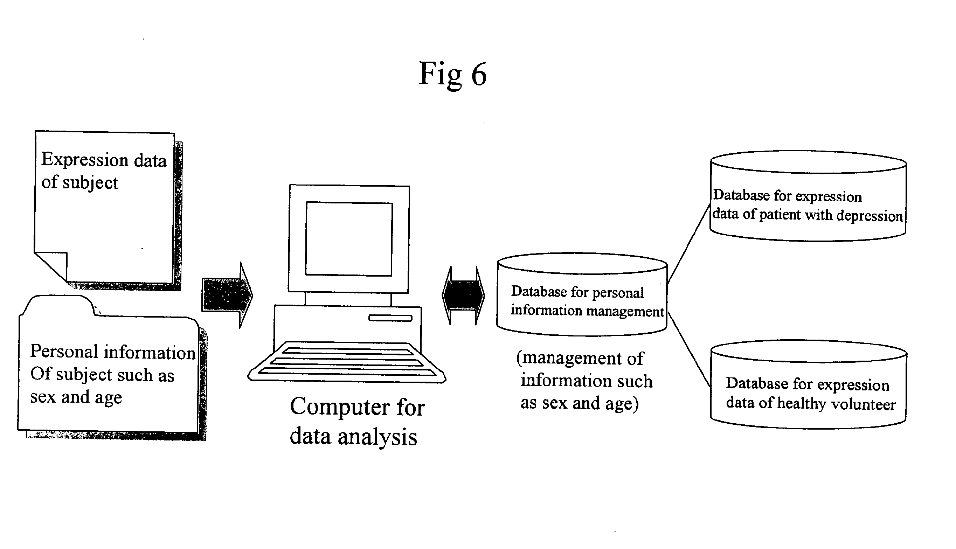

The present invention has been completed based on the results of above experimentation. In the present invention, mRNA is extracted from a subject's peripheral blood, and its expression profile is examined, thereby resulting in diagnosis of depression in the subject in accordance with the type of depression. Fig. 5 schematically shows the method of diagnosing depression of the present invention, and Fig. 6 schematically shows the system of diagnosing depression of the present invention.

Techniques for examining the gene expression levels employed in the present invention are not limited to the DNA chips shown in Fig. 5. Any conventional techniques for analysis in the art can be employed. For example, nucleic acid hybridization utilizing other DNA-immobilized solid substrates such as DNA arrays or membrane filters, quantitative PCR such as RT-PCR or real-time PCR, Northern blotting, subtraction, differential display, differential hybridization, and cross-hybridization, can be employed. DNA-immobilized solid substrates, such as DNA chips, DNA arrays, membrane filters, and capillaries, are particularly preferable since a large number of genes can be extensively analyzed at a single operation.

The solid substrate that is employed in the present invention is prepared by immobilizing probes that each independently specifically hybridize to any one of the genes listed in Tables 1 to 4 to detect the target gene on a solid substrate, such as a glass or nylon membrane. Preferably, the target genes to be immobilized on the substrate at least include ATP2A2, SCYA5, STIP1, EEF1A1, GRB10, CASP6, TSSC1, RAB9, NFATC3, and TPR listed in Table 1, GNG10, CLK1, P2Y5, IFNGR1, TAF2F, PIM1, MAP2K3, HDGF, INSR, and COX6C listed in Table 2, CDC10, GZMA, TNFRSF6, HSPCA, NR3C1, TOPBP1, ARNTL, RAP1A, POLR2B, and ITGB1 listed in Table 3, and POU2F2, BCL2L1, DAXX, COX4, CD3G, FCER1G, NME2, CPT1B, HSPE1, and COX7A2 listed in Table 4. Alternatively, the solid substrate of the present invention is prepared by immobilizing probes that each independently specifically hybridize to any one of the genes listed in Tables 7 to 10 to detect the target gene on a solid substrate, such as a glasses or nylon membrane. Preferably, the target genes to be immobilized on the substrate at least include HLA-G, HRH4, PSMB9, ATP2A2, SCYA5, SLC6A4, CASP6, CSF2, HSD3B1, and RAB9 listed in Table 7, HSPE1, PSMA4, ADH5, PSMA6, COX17, HMG1, GPR24, COX6C, FGF2, and COX7C listed in Table 8, CLK1, PSMC6, TAF2F, P2Y5, CASP3, HSPCA, MSH2, SLC38A2, B2M, and AKAP11 listed in Table 9, and CCNA2, HGF, GPR24, PTGER3, COX7A2, BDKRB2, UFD1L, HMG1, PSMA4, and ATP6J listed in Table 10. A probe that is employed to detect genes can be designed as a sequence that is complementary to a region with high specificity of the marker gene (e.g., 3' UTR) in accordance with a conventional technique. A synthetic oligo probe with a 25-100 base length or a PCR product with a 300-1,000 base length can be employed. A method of immobilizing a probe on a solid substrate is not particularly limited. In accordance with a conventional technique, a synthesized probe may be spotted on a solid substrate or a probe may be synthesized on a solid substrate.

For example, the RNA sample collected from a subject and the RNA sample collected from a healthy volunteer are respectively labeled with fluorescent dyes having different emission wavelengths, and they are applied to the same DNA chip for diagnosing depression to conduct competitive hybridization. The fluorescence intensity of each probe on the chip represents the differences in the gene expression intensities between the subject and the healthy volunteer. The expression profiles thereof can be then examined to diagnose the conditions of depression in the subject.

Alternatively, a certain RNA sample, for example, a commercialized universal RNA sample, is used as a standard sample, and comparison and analysis of expression levels of the subject's sample and the standard sample are conducted separately from those of the healthy volunteer's sample and the standard sample in the aforementioned manner to analyze expression data for both groups in comparison with each other. Thus, the conditions of depression in the subject can be diagnosed.

In any case, a subject and a healthy volunteer to be compared therewith preferably have the same age and sex conditions. For example, an acceptable age gap between them is up to 5 years.

If the expression data for healthy volunteers are classified in accordance with their age and sex and stored in a database, the subject and a healthy volunteer can be compared and analyzed by simply retrieving the data that match the conditions of the subject in terms of age and sex from the database. Also, the expression data for patients afflicted with depression and those for healthy volunteers are previously stored in the computer, and the computer is allowed to determine which of the expression patterns for patients or healthy volunteers are more similar to the subject's expression data, thereby diagnosing the conditions of depression in the subject (see Fig. 6).

Further, if the expression data for patients afflicted with depression is stored in the computer in accordance with the group (the PA group and the PB group), more accurate diagnosis in accordance with the type of depression in the subject can be realized. In accordance with the expression data of each group stored in the computer, for example, the computer is allowed to determine which of the expression patterns are more similar to those of the subject who had been diagnosed as afflicted with depression, and the evaluated data is then clustered. The clustered data of the subject is further evaluated by the computer in terms of the conditions or the course of treatment based on the expression profile of a diagnostic marker specific for each group.

A method for data analysis is not limited to clustering. Any conventional analytical techniques in the art, for example, a machine learning algorithm such as the one utilizing a support vector machine can be employed.

The method of the present invention can conduct the analysis with the use of 5 ml of blood obtained by conventional blood sampling without special cooperation provided by a patient. This diagnostic method can be carried out in a non-invasive, simple, and routine manner. This method of multidimensionally comprehending biological functions based on numerous mRNA expression levels is more adequate as a method of diagnosing complicated psychiatric diseases involving both mental and physical conditions such as depression in terms of its principle compared with the conventional method that assays only limited factors.

The results attained by the method of the present invention can be simply and clearly evaluated, they can be easily employed by primary care doctors as objective indicators for depression, and they are extremely useful for the establishment of diagnosis and introduction of therapy. A high-risk group can be accurately selected from among the groups of people through medical checkups or complete physical examinations provided by workplaces, schools, and communities. This enables early detection of depression in a simple and cost-effective manner. Accordingly, the method of the present invention significantly contributes to the improvement of peoples' mental health from the viewpoint of preventive care.

The usefulness of the method according to the present invention is not limited to primary care and medical checkups. Specialists in psychiatric medicine can apply this technique to the search for psychological, social, and environmental factors associated with the development of depression, evaluation of clinical conditions, diagnosis, evaluation of treatment, and determination of prognosis. Thus, this technique can be a revolutionary test technique in the field of psychiatric medicine, which dramatically improves a technique of diagnosing depression.

The present invention is hereafter described in greater detail with reference to the following examples, although it is not limited to these examples.

### [Example 1] Selection of marker gene

### 1. Patients and healthy volunteers

Target patients were those who had agreed with the written description for participating in the research for developing the present diagnostic method selected from among untreated patients afflicted with depression who had visited the Department of Psychiatry and Neurology of the Tokushima University Hospital between November 2001 and June 2002. This research was approved by the ethics committee of Tokushima University Hospital. Diagnosis was made in accordance with a depressive episode specified in the International Classification of Diseases, 10th revision (ICD-10). Patients with serious physical complications or those taking therapeutic agents for physical diseases were excluded. Healthy volunteers with the same sex and age conditions were selected for each patient for comparison.

Thirty three patients whose samples before treatment had been obtained were 25 males and 8 females aged 23 to 74 (45.7 years old on average), and their Hamilton scores were between 10 and 38 points (23.2 points on average).

Samples were obtained from 15 patients after the treatment. They were 13 males and 2 females aged 27 to 68 (48.1 years old on average), and their Hamilton scores were between 2 and 25 (6.9 points on average). Treatment was mainly carried out by medication using antidepressants. The remission of symptoms was determined based on general clinical diagnosis. Samples satisfied the standard of having scores of 7 or less on the Hamilton Rating Scale, which are generally regarded as representing remission of symptoms, except for 5 samples. Samples after treatment were collected 68 to 211 days after the collection of samples before treatment (121 days on average). The mRNA expression level after treatment was compared with that of a sample taken from the same subject before treatment.

### 2. Analysis of gene expression

Blood (5 ml) was collected from the patients, and total RNA was extracted using a PAXgene Blood RNA System (Qiagen). Blood was collected by a doctor or nurse between 10:00 am and 1:00 pm from the patients under fasting conditions through cubitus veins under resting conditions. The yield of total RNA was 5 µg to 15 µg.

Subsequently, 5 µg of total RNA extracted from each patient was separated, annealed with an oligo (dT) 24 primer comprising a T7 promoter sequence added thereto, and first-strand DNA was synthesized. Thereafter, this first-strand DNA was used as a template to synthesize second-strand DNA having a T7 promoter sequence. Finally, the second-strand DNA was used as a template to synthesize RNA with the aid of T7 RNA polymerase. A random hexamer was annealed to 6 µg of the synthesized RNA to conduct a reverse transcriptase reaction, and Cy5-dCTP was incorporated into the strand. Thus, fluorescence-labeled cDNA was synthesized.

In a manner similar to the case of the patients, 5 ml of blood was collected from each of 33 healthy volunteers with the same sex and age conditions, and total RNA was then extracted. cDNA was similarly synthesized except for the use of Cy3 as a fluorescent label.

When comparing samples of a single subject before and after treatment, cDNA labeled with Cy3 and cDNA labeled with Cy5 were synthesized from the samples before and after treatment, respectively.

Equivalent amounts of two types of cDNAs for comparison and analysis were mixed, the resultant was applied to a DNA chip (a DNA chip for analyzing drug response, Hitachi Co., Ltd.), and hybridization was carried out at 62°C for 12 hours. After washing, fluorescence intensity at each spot was assayed using a scanner (ScanArray 5000, GSI-Lumonics). Differences in gene expression levels between samples obtained from patients and samples obtained from healthy volunteers or those between samples obtained from a single patient before and after treatment were determined.

### 3. Data analysis

### (1) Selection of marker gene for depression

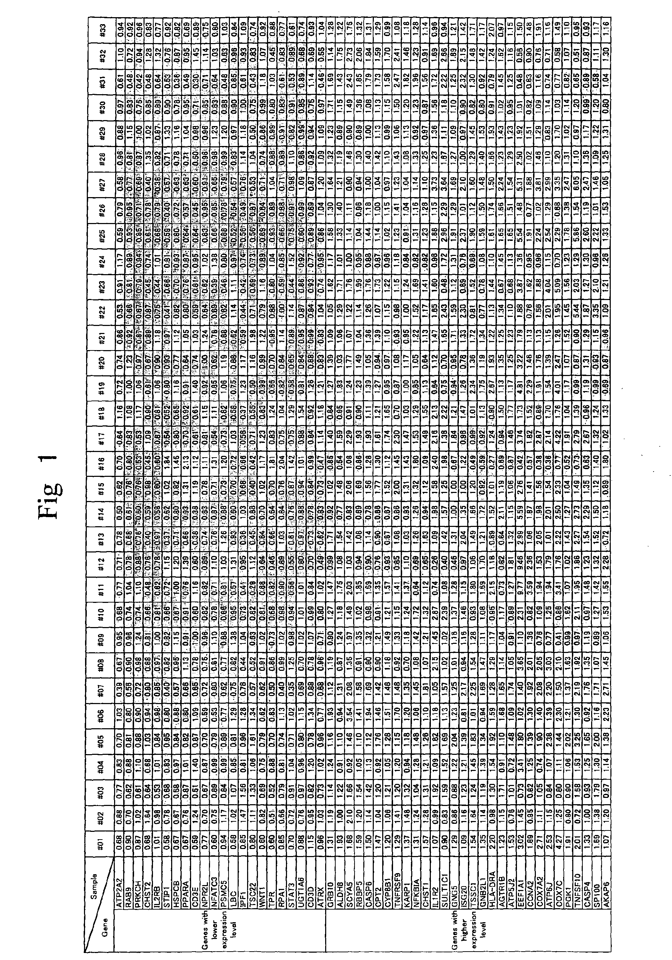

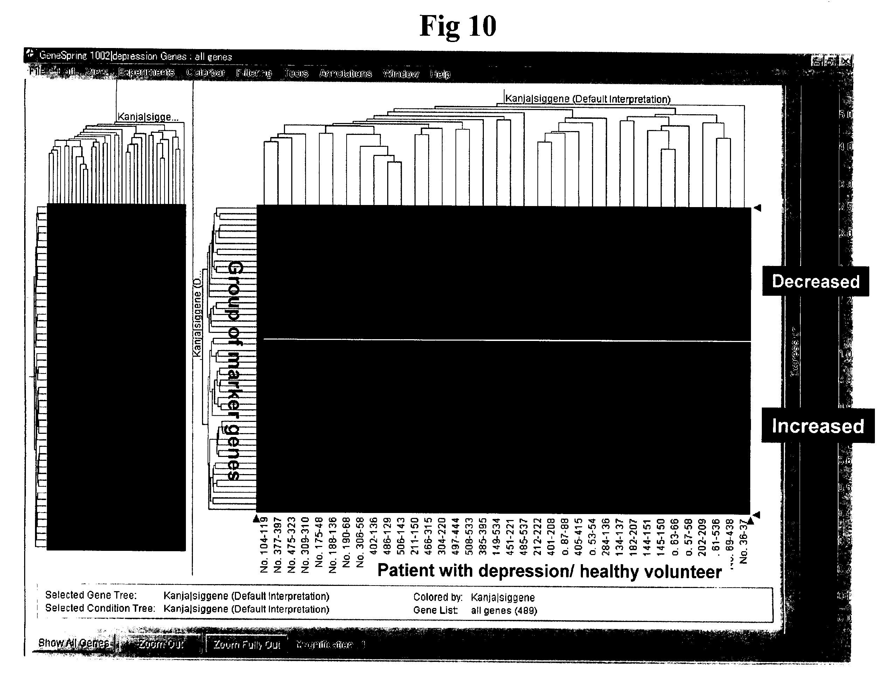

A group of genes (489 genes) having fluorescence intensities of 300 or higher in all 48 groups of data was selected as the object of analysis. Among the data on patient/healthy volunteer comparison, the gene with a significantly higher or lower expression level was selected via a significant difference test. There were 30 genes of the patient with a significantly higher expression level compared to that of the healthy volunteer and 22 genes thereof with a significantly lower expression level (Fig. 1, Fig. 10, Table 1). These 52 genes are useful for evaluating whether or not the subject has been afflicted with depression, i.e., they are useful as marker genes for depression. Among them, the expression levels of ATP2A2, SCYA5, STIP1, EEF1A1, GRB10, CASP6, TSSC1, RAB9, NFATC3, and TPR were significantly varied, and thus, they were considered to be particularly useful marker genes for depression.

**Table 1:**

| Group of genes exhibiting significant differences between patient/healthy volunteer | | | |
|---|---|---|---|
| Symbol | Name | Category | GenBank ID |
| AGTR1B | H.sapiens mRNA for angiotensin II receptor | angiotensin | X65699 |
| AKAP6 | Homo sapiens A kinase (PRKA) anchor protein 6 (AKAP6) | Signal | NM_004274 |
| ALDH8 | Human aldehyde dehydrogenase (ALDH8) mRNA | ALDH | U37519 |
| ATP2A2 | ATPase, Ca++ transporting, cardiac muscle, slow twitch 2 | ATPase | M23114 |
| ATP5J2 | ATP synthase, H+ transporting, mitochondrial F0 complex, subunit f, isoform 2 | ATPase | AF047436 |
| ATP6J | ATPase, H+ transporting, lysosomal (vacuolar proton pump), member J | ATPase | AF038954 |
| ATRX | Alpha thalassemia/mental retardation syndrome X-linked | ATPase | U72938 |
| CASP4 | Human cysteine protease (ICErel-II) mRNA, complete cds | Appoptosis | U28014 |
| CASP6 | Human cysteine protease Mch2 isoform alpha (Mch2) mRNA, complete cds | Appoptosis, Signal | U20536 |
| CCNA2 | Human mRNA for cyclin A; Cyclin A2 | CeliCycle | X51688 |
| CD3D | Homo sapiens CD3D antigen, delta polypeptide (TiT3 complex) (CD3D), mRNA | Signal | NM_000732 |
| CD3E | Human mRNA for T3 epsilon chain (20K) of T-cell receptor (from peripheral blood lymphocytes). | Signal | X03884 |
| CHST1 | Homo sapiens mRNA for keratan sulfate Gal-6-sulfotransferase | sulfotransferase | AB003791 |
| CHST2 | Homo sapiens carbohydrate (N-acetylglucosamine-6-O) sulfotransferase 2 (CHST2) | sulfotransferase | NM_004267 |
| COX7A2 | Homo sapiens cytochrome c oxidase subunit VIIa polypeptide 2 (liver)(COX7A2), nuclear gene encoding mitochondrial protein | mitochondria & stress | NM_001865 |
| COX7C | Homo sapiens cytochrome c oxidase subunit VIIc | mitochondria & stress | NM_001867 |
| CPT2 | Homo sapiens camitine palmitoyltransferase II (CPT2), nuclear gene encoding mitochondrial protein | mitochondria & stress | NM_000098 |
| CYP8B1 | Homo sapiens sterol 12-alpha hydroxylase CYP8B1 (Cyp8b1) mRNA,partial cds | P450 | AF090318 |
| EEF1A1 | Homo sapiens eukaryotic translation elongation factor 1 alpha 1 (EEF1A1) | glucocorticoids (Cortisol) | NM_001402 |
| GNB2L1 | Human MHC protein homologous to chicken B complex protein mRNA; Guanine nucleotide binding protein (G protein), beta polypeptide 2-like 1 | Signal | M24194 |
| GNG5 | Homo sapiens G protein gamma 5 subunit mRNA; Guanine nucleotide binding protein (G protein), gamma 5 | Signal | AF038955 |
| GRB10 | Homo sapiens growth factor receptor-bound protein 10 (GRB10), mRNA | Insulin | NM_005311 |
| HLA-DRA | Human HLA-DR alpha-chain mRNA; Class II MHC alpha | Signal | K01171 |
| HSPCB | Human 90-kDa heat-shock protein gene, cDNA; Heat shock 90kD protein 1, beta | hsp | M16660 |
| IL1R2 | H.sapiens IL-1 R2 mRNA for type II interleukin-1 receptor, (cell line CB23). | Cytokine | X59770 |
| IL2RB | Human interleukin 2 receptor beta chain (p70-75) mRNA, complete cds | Cytokine, Signal | M26062 |
| IPF1 | Homo sapiens insulin promoter factor 1, homeodomain transcription factor (IPF1) | Insulin | NM_000209 |
| ISG20 | Human HEM45 mRNA, complete cds | Cytokine | U88964 |
| KARP1 | Ku86 autoantigen related protein 1 | Signal | AF039597 |
| LBC | Human P47 LBC oncogene mRNA, complete cds | oncogene | U03634 |
| NFATC3 | Homo sapiens NF-AT4c mRNA, complete cds | Signal, TF | L41067 |
| NFKBIA | Homo sapiens MAD-3 mRNA encoding IkB-like activity, complete cds, IkBalpha | Signal | M69043 |
| NPR2L | Homo sapiens candidate tumor suppressor gene 21 protein mRNA, complete cds | Supressor | AF040708 |
| PGK1 | phosphoglycerate kinase 1 | polymerase | V00572 |
| PPARA | Human peroxisome proliferator activated receptor mRNA, complete cds | PPAR | L02932 |
| PRKCH | Human protein kinase C-L (PRKCL) mRNA; Protein kinase C, eta | Signal | M55284 |
| PSMC5 | Proteasome (prosome, macropain) 26S subunit, ATPase, 5 | ATPase | AF035309 |
| RAB9 | Human small GTP binding protein Rab9 mRNA, complete cds. | oncogene | U44103 |
| RBBP5 | H.sapiens RBQ-3 mRNA | Signal | X85134 |
| RPA1 | Replication protein A1 (70kD) | Signal | M63488 |
| SCYA5 | Human T cell-specific protein (RANTES) mRNA, Small inducible cytokine A5 | Cytokine | M21121 |
| SP100 | Human nuclear autoantigen (SP-100) mRNA | Signal | M60618 |
| STAT3 | Homo sapiens DNA-binding protein (APRF) mRNA, complete cds | Signal, TF | L29277 |
| STIP1 | Homo sapiens stress-induced-phosphoprotein 1 (Hsp70/Hsp90-organizing protein) | stress | NM_006819 |
| SULT1C1 | Human sulfotransferase mRNA family 1C, member 1 (SULT1C1) | sulfotransferase | U66036 |
| TNFRSF9 | Human activation dependent T cell mRNA, complete cds | Cytokine | L12964 |
| TNFSF10 | Human TNF-related apoptosis inducing ligand TRAIL mRNA, complete cds | Cytokine | U37518 |
| TPR | H.sapiens tpr mRNA; Translocated promoter region (to activated MET oncogene) | oncogene | X66397 |
| TSC22 | Human putative regulatory protein TGF-beta-stimulated clone 22 homolog (TSC22) | GF | U35048 |
| TSSC1 | Homo sapiens tumor suppressing STF cDNA 1 (TSSC1) mRNA, complete cds | Supressor | AF019952 |
| UGT1 A6 | Homo sapiens phenol UDP-glucuronosyltransferase (UDPGT) mRNA | UGT | J04093 |
| WNT1 | Homo sapiens wingless-type MMTV integration site family, member 1 (WNT1), mRNA | oncogene, Signal | NM_005430 |

### (2) Selection of marker gene for classification

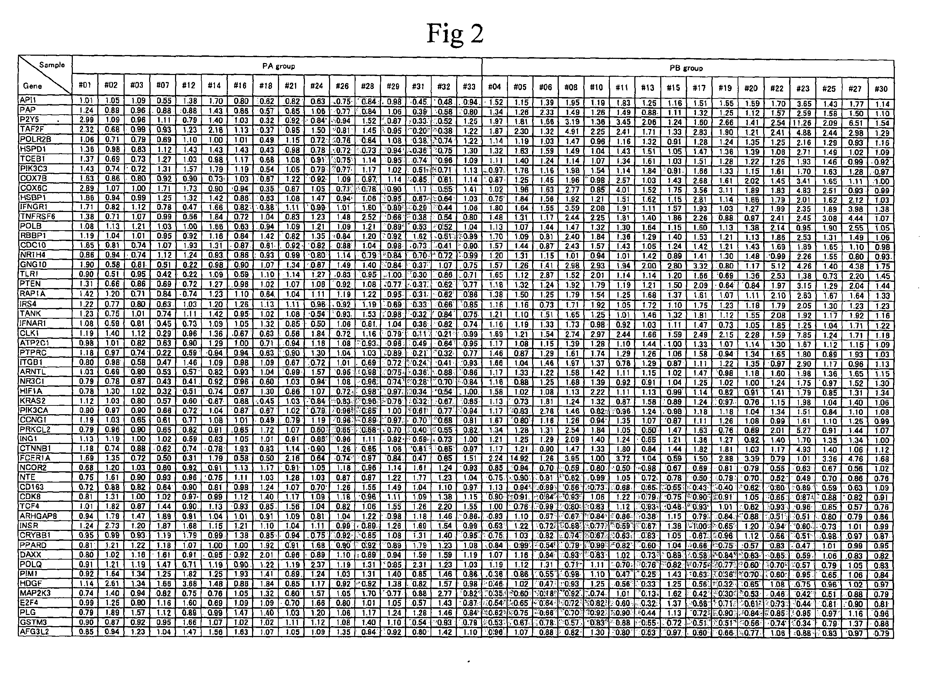

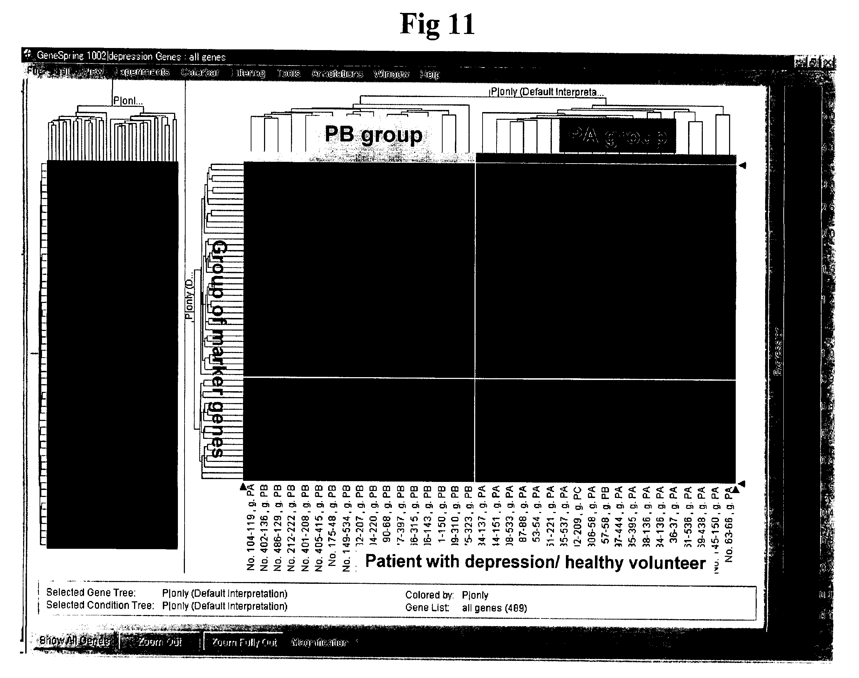

Thirty three pairs of subjects for patient/healthy volunteer comparison were subjected to cluster analysis utilizing all the genes (489 genes). Analysis was carried out by hierarchical clustering based on the cosine coefficient distance without a weight between clusters. This cluster analysis demonstrated that the patient/healthy volunteer comparison samples were roughly divided into 2 groups. Such 2 groups were designated as the PA group and the PB group. The 33 pairs of subjects for patient/healthy volunteer comparison were divided into the PA group (16 pairs), the PB group (16 pairs), and a pair that did not belong to either group. In order to extract the genes that were peculiar to the PA group and to the PB group, these groups were compared to each other. There were 56 genes that exhibited significant differences between the PA group and the PB group (Fig. 2, Fig. 11, Table 2). These 56 genes are useful for assigning patients afflicted with depression to the PA or PB group, i.e., they are useful as marker genes for classification the patients afflicted with depression. Among them, the expression levels of GNG10, CLK1, P2Y5, IFNGR1, TAF2F, PIM1, MAP2K3, HDGF, INSR, and COX6C were significantly varied, and thus, they were considered to be particularly useful marker genes for classification (Table 4).

**Table 2:**

| Genes exhibiting significant differences between PA group and PB group | | | |
|---|---|---|---|
| Symbol | Name | Category | GenBank ID |
| AFG3L2 | AFG3 (ATPase family gene 3, yeast)-like 2 | ATPase | NM_006796 |
| API1 | Human inhibitor of apoptosis protein 2 mRNA; Apoptosis inhibitor 1 | Appoptosis, Signal | U45879 |
| ARHGAP8 | Homo sapiens Rho GTPase activating protein 8 (ARHGAP8), mRNA | Signal | NM_015366 |
| ARNTL | Homo sapiens mRNA for BMAL1a; aryl hydrocarbon receptor nucleartranslocator-like | Ah receptor | D89722 |
| ATP2C1 | ATPase, Ca++-sequestering | ATPase | AF225981 |
| CCNG1 | Human cyclin G1 mRNA, complete cds | CellCycle | U47413 |
| CD163 | Homo sapiens CD163 antigen (CD163) | expressed exclusively on human monocyte; glucocorticoid-inducibl e | NM_004244 |
| CDC10 | hCDC10=CDC10 homolog [human, fetal lung, mRNA, 2314 nt]. | CellCycle | S72008 |
| CDK8 | Homo sapiens mRNA for CDK8 protein kinase. | CellCycle | X85753 |
| CLK1 | Homo sapiens clk1 mRNA; CDC-like kinase 1 | CellCycle | L29222 |
| COX6C | Homo sapiens cytochrome c oxidase subunit VIc (COX6C), nuclear gene encoding mitochondrial protein | mitochondria & stress | NM_004374 |
| COX7B | Homo sapiens cytochrome c oxidase subunit VIIb | mitochondria & stress | NM_001866 |
| CRYBB1 | Human beta B1-crystallin mRNA | sulfotransferase | U35340 |
| CTNNB1 | H.sapiens mRNA for beta-catenin | Signal | X87838 |
| DAXX | Homo sapiens Fas-binding protein Daxx mRNA, complete cds | Signal | AF015956 |
| E2F4 | Homo sapiens E2F transcription factor 4, p107/p130-binding (E2F4) | TF | NM_001950 |
| FCER1A | Human mRNA for high affinity IgE receptor alpha-subunit (FeERI); Fc fragment of IgE, high affinity I, receptor for, alpha polypeptide. | Signal | X06948 |
| GNG10 | Human G protein gamma-10 subunit mRNA; Guanine nucleotide binding protein 10 | Signal | U31383 |
| GSTM3 | Human glutathione transferase M3 (GSTM3) mRNA | GSTM | J05459 |
| HDGF | Human mRNA for hepatoma-derived growth factor, complete cds | GF | D16431 |
| HIF1A | Homo sapiens hypoxia-inducible factor 1, alpha subunit (basic helix-loop-helix transcription factor) | hypoxia, TF | NM_001530 |
| HSBP1 | Homo sapiens heat shock factor binding protein 1 HSBP1 mRNA; Heat shock factor binding protein 1 | hsp | AF068754 |
| HSPD1 | Heat shock 60kD protein 1 (chaperonin) | hsp | M34664 |
| IFNAR1 | Human interferon-alpha receptor (HuIFN-alpha-Rec) mRNA, complete cds | Cytokine, Signal | J03171 |
| IFNGR1 | Human interferon-gamma receptor mRNA, complete cds | Cytokine, Signal | J03143 |
| ING1 | Homo sapiens growth inhibitor p33ING1 (ING1) mRNA, complete cds | Signal, Supressor | AF001954 |
| INSR | Homo sapiens insulin receptor (INSR), mRNA. | Insulin | NM_000208 |
| IRS4 | Homo sapiens insulin receptor substrate 4 (IRS4) | Insulin | NM_003604 |
| ITGB1 | Integrin, beta 1 (fibronectin receptor, beta polypeptide, antigen CD29 includes MDF2, MSK12); | Signal | X07979 |
| KRAS2 | Human K-ras oncogene protein mRNA (KRAS2) | oncogene | M54968 |
| MAP2K3 | Human mRNA for MAP kinase kinase 3b complete cds, MEK3 | Signal | D87116 |
| NCOR2 | Human silencing mediator of retinoid and thyroid hormone action (SMRT) mRNA, Nuclear receptor co-repressor 2 | NR | U37146 |
| NR1H4 | Human famesol receptor HRR-1 (HRR-1) mRNA, complete cds | NR1(FXR) | U68233 |
| NR3C1 | Human glucocorticoid receptor alpha mRNA, complete cds | glucocorticoids (Cortisol) | M10901 |
| NTE | Homo sapiens mRNA for neuropathy target esterase | esterase | AJ004832 |
| P2Y5 | Homo sapiens purinergic receptor P2Y5 mRNA | Signal | AF000546 |
| PAP | poly(A) polymerase | polymerase | X76770 |
| PIK3C3 | H.sapiens mRNA for phosphatidylinositol 3-kinase, Phosphoinositide-3-kinase, class 3 | Signal | Z46973 |
| PIK3CA | Human phosphoinositide 3'-hydroxykinase p110-alpha subunit mRNA, Phosphoinositide-3-kinase, catalytic, alpha polypeptide | Signal | U79143 |
| PIM1 | Human h-pim-1 protein (h-pim-1) mRNA, complete cds | oncogene | M54915 |
| PLG | Human mRNA for plasminogen | Signal | X05199 |
| POLB | polymerase (DNA directed), beta | polymerase | D29013 |
| POLQ | polymerase (DNA-directed), theta | polymerase | AF043628 |
| POLR2B | polymerase (RNA) II (DNA directed) polypeptide B (140kD) | polymerase | X63563 |
| PPARD | Human peroxisome proliferator activated receptor mRNA, complete cds | PPAR | L07592 |
| PRKCL2 | Human lipid-activated, protein kinase PRK2 mRNA; Protein kinase C-like 2 | Signal | U33052 |
| PTEN | Human mutated in multiple advanced cancers protein (MMAC1) mRNA; putative protein-tyrosine phosphatase PTEN | Supressor | U92436 |
| PTPRC | Human mRNA for T200 leukocyte common antigen (CD45, LC-A). | Signal | Y00062 |
| RAP1A | Human ras-related protein (Krev-1) mRNA, complete cds | Supressor | M22995 |
| RBBP1 | Homo sapiens retinoblastoma-binding protein 1 (RBBP1) mRNA | Signal | NM_002892 |
| TAF2F | TATA box binding protein (TBP)-associated factor, RNA polymerase II, F, | polymerase, TF | U18062 |
| | 55kD | | |
| TANK | Human TRAF family member-associated NF-kB activator TANK mRNA, I-TRAF | Signal | U63830 |
| TCEB1 | transcription elongation factor B (SIII), polypeptide 1 (15kD, elongin C) | polymerase, TF | L34587 |
| TCF4 | Homo sapiens transcription factor 4 (TCF4) | Signal, TF | NM_003199 |
| TLR1 | Homo sapiens Toll-like receptor 1 (TLR1) mRNA, complete cds | Signal | U88540 |
| TNFRSF6 | H.sapiens mRNA for APO-1 cell surface antigen, FAS | Appoptosis, Cytokine, Signal | X63717 |

### (3) Selection of diagnostic marker gene for each group

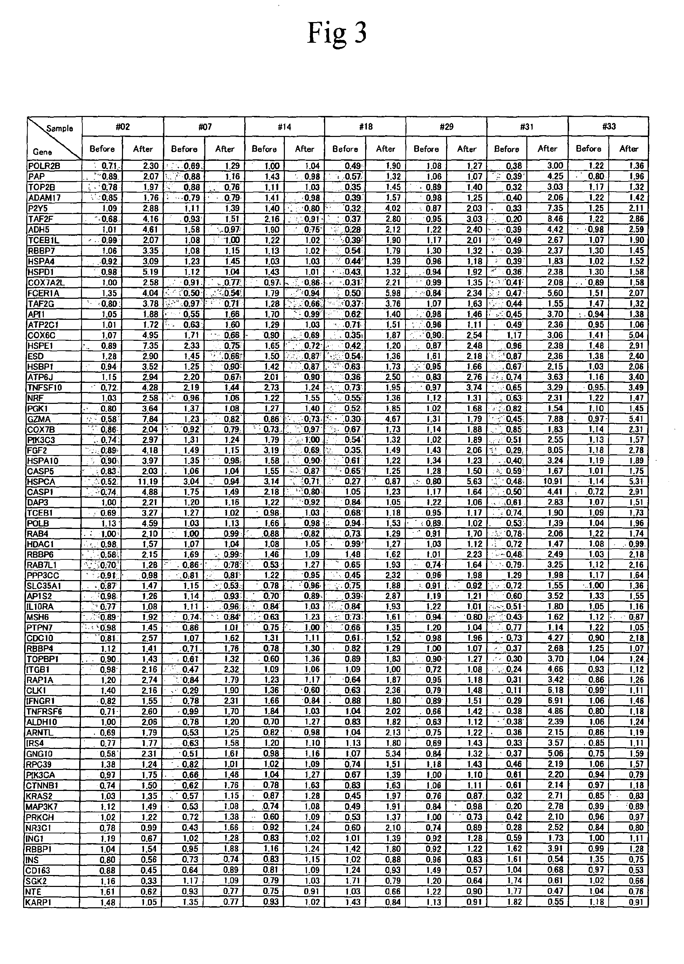

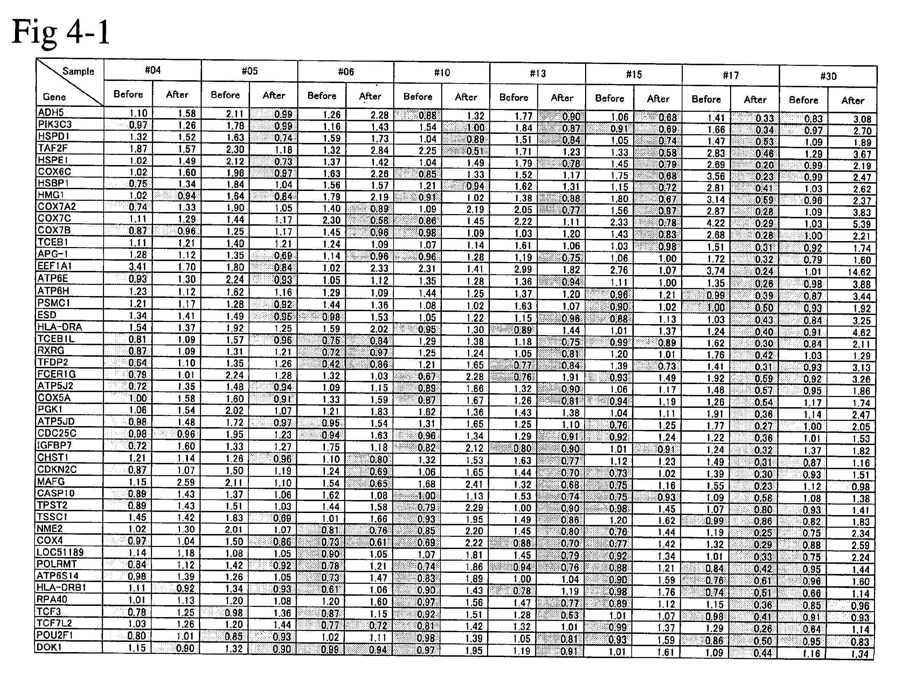

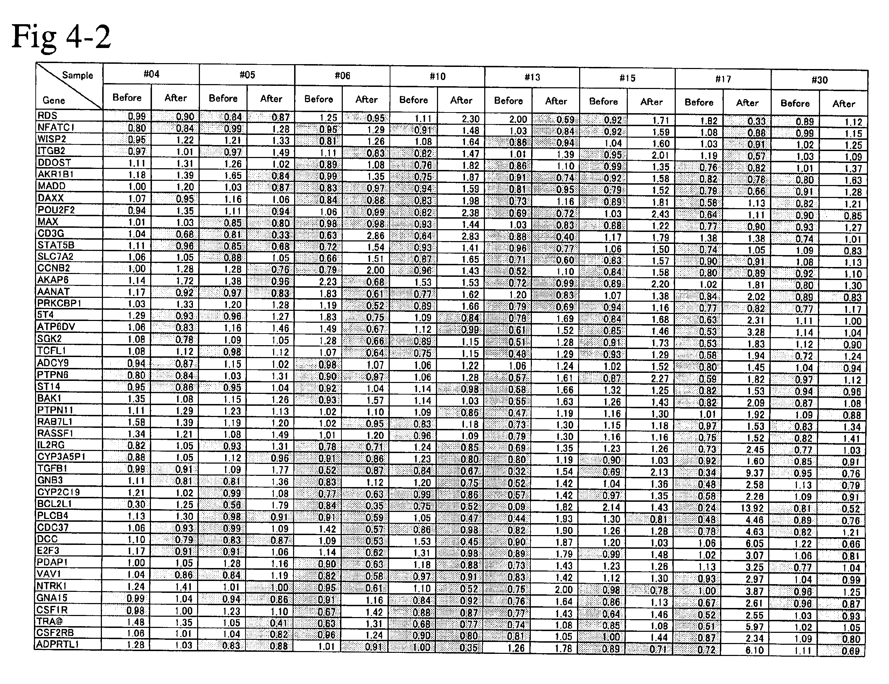

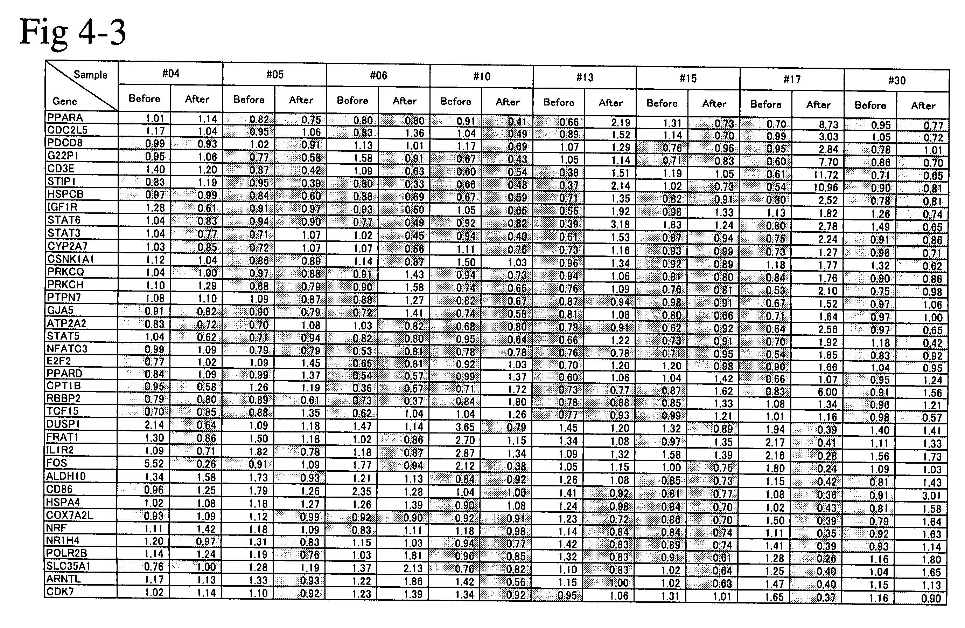

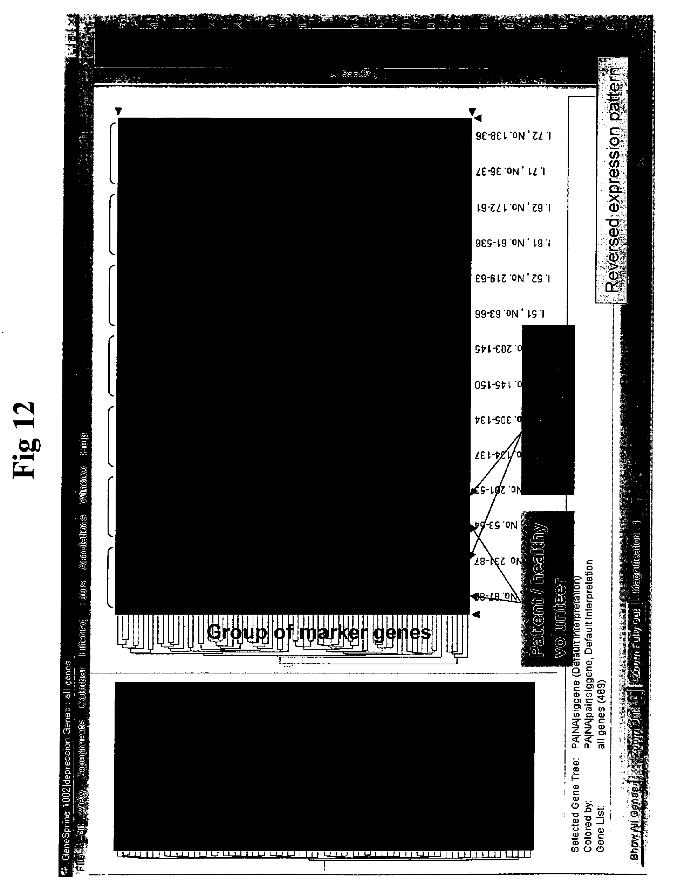

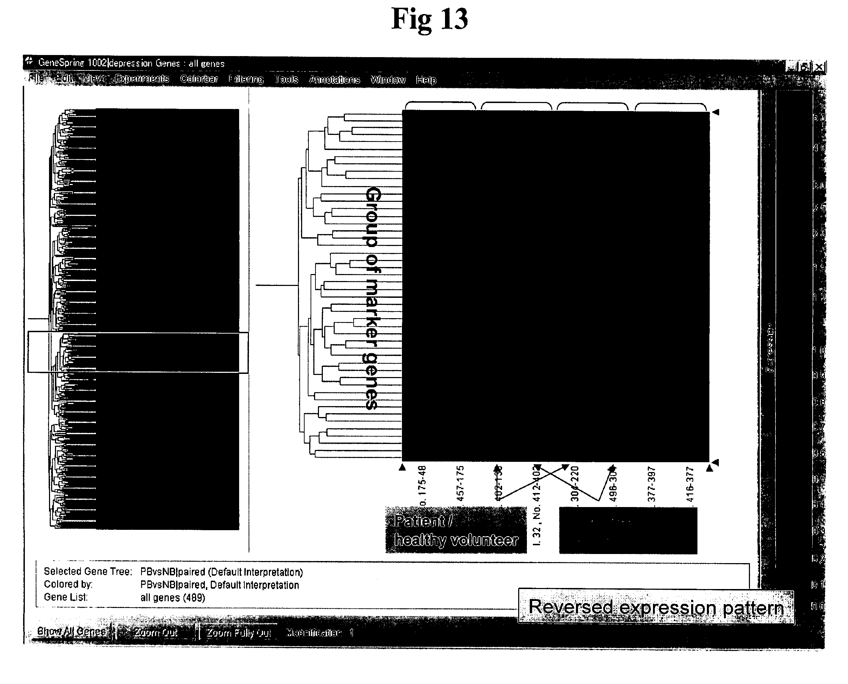

Based on the results attained above, 15 subjects for before/after treatment comparison were divided into the PA group (7 subjects) and the PB group (8 subjects). The data on patient/healthy volunteer comparison and the data on before/after treatment comparison were aligned for each patient in each group, and the data were compared and analyzed. The group of genes with reversed expression patterns between the patient/healthy volunteer comparison sample and the before/after treatment comparison sample was extracted (PA group: Fig. 3, Fig. 12, Table 3; PB group: Fig. 4 Fig. 13, Table 4). Concerning the PA group, variations in expression levels of CDC10, GZMA, TNFRSF6, HSPCA, NR3C1, TOPBP1, ARNTL, RAP1A, POLR2B, and ITGB1 were particularly significant among the genes listed in Table 3. Concerning the PB group, variations in expression levels of POU2F2, BCL2L1, DAXX, COX4, CD3G, FCER1G, NME2, CPT1B, HSPE1, and COX7A2 were particularly significant among the genes listed in Table 4.

Changes in the Hamilton scores before and after the treatment are shown in Table 5. The reversed expression patterns between the data on patient/healthy volunteer comparison and the data on before/after treatment comparison indicate a change in gene expression that is observed characteristically when the patient afflicted with depression received treatment involving the use of an antidepressant. The group of genes is useful as an indicator for the conditions or the course of treatment of the patients afflicted with depression in each group. Specifically, they are useful diagnostic marker genes that are specific for each group.

**Table 3:**

| Genes exhibiting significant differences before and after treatment in PA group | | | |
|---|---|---|---|
| Symbol | Name | Category | GenBank ID |
| ADAM17 | Homo sapiens snake venom-like protease (cSVP) mRNA. A disintegrin and metalloproteinase domain 17 (tumor necrosis factor, alpha, converting enzyme) | Cytokine | U92649 |
| ADH5 | Human alcohol dehydrogenase class III (ADH5) mRNA | ADH | M29872 |
| ALDH10 | Human microsomal aldehyde dehydrogenase (ALD10) mRNA | ALDH | U46689 |
| AP1S2 | Homo sapiens adaptor-related protein complex 1, sigma 2 subunit (AP1S2) | AP-1 | NM_003916 |
| API1 | Human inhibitor of apoptosis protein 2 mRNA; Apoptosis inhibitor 1 | Appoptosis, Signal | U45879 |
| ARNTL | Homo sapiens mRNA for BMAL1a; aryl hydrocarbon receptor nuclear translocator-like | Ah receptor | D89722 |
| ATP2C1 | ATPase, Ca++-sequestering | ATPase | AF225981 |
| ATP6J | ATPase, H+ transporting, lysosomal (vacuolar proton pump), member J | ATPase | AF038954 |
| CASP1 | Human interleukin 1-beta converting enzyme isoform delta (IL1BCE) mRNA, complete cds | Appoptosis, Signal | U13699 |
| CASP5 | Human cysteine protease (ICErel-III) mRNA, complete cds | Appoptosis | U28015 |
| CD163 | Homo sapiens CD163 antigen (CD163) | expressed exclusively on human monocyte; glucocorticoid-ind ucible | NM_004244 |
| CDC10 | hCDC10=CDC10 homolog [human, fetal lung, mRNA, 2314 nt]. | CellCycle | S72008 |
| CLK1 | Homo sapiens clk1 mRNA; CDC-like kinase 1 | CellCycle | L29222 |
| COX6C | Homo sapiens cytochrome c oxidase subunit VIc (COX6C), nuclear gene encoding mitochondrial protein | mitochondria stress | & NM_004374 |
| COX7A2L | Homo sapiens cytochrome c oxidase subunit VIIa polypeptide 2 like | mitochondria & stress | NM_004718 |
| COX7B | Homo sapiens cytochrome c oxidase subunit VIIb | mitochondria & stress | NM_001866 |
| CTNNB1 | H.sapiens mRNA for beta-catenin | Signal | X87838 |
| DAP3 | Human ionizing radiation resistance conferring protein mRNA; Death associated protein 3 | Appoptosis | U18321 |
| ESD | Homo sapiens esterase D mRNA | esterase | AF112219 |
| FCER1A | Human mRNA for high affinity IgE receptor alpha-subunit (FcERI); Fc fragment of IgE, high affinity I, receptor for; alpha polypeptide | Signal | X06948 |
| FGF2 | Human basic fibroblast growth factor (FGF) mRNA (BFGF; FGFB; FGF2) | GF | M27968 |
| GNG10 | Human G protein gamma-10 subunit mRNA; Guanine nucleotide binding protein 10 | Signal | U31383 |
| GZMA | Human Hanukah factor serine protease (HuHF) mRNA (cytotoxic T-lymphocyte-associated serine esterase 3) | esterase | M18737 |
| HDAC1 | Human mRNA for RPD3 protein, Histone deacetylase 1 | Signal, TF | D50405 |
| HSBP1 | Homo sapiens heat shock factor binding protein 1 HSBP1 mRNA; Heat shock factor binding protein 1 | hsp | AF068754 |
| HSPA10 | Homo sapiens heat shock 70kD protein 10 (HSC71) (HSPA10), mRNA | hsp | NM_006597 |
| HSPA4 | Human heat shock protein 70 (hsp70) mRNA; Heat shock 70kD protein 4 | hsp | L12723 |
| HSPCA | Homo sapiens Hsp89-alpha-delta-N mRNA; Heat shock 90kD protein 1, alpha | hsp | AF028832 |
| HSPD1 | Heat shock 60kD protein 1 (chaperonin) | hsp | M34664 |
| HSPE1 | Human chaperonin 10 mRNA; Heat shock 10kD protein 1 | hsp | U07550 |
| IFNGR1 | Human interferon-gamma receptor mRNA, complete cds | Cytokine, Signal | J03143 |
| IL10RA | Human interleukin-10 receptor mRNA, complete cds | Cytokine | U00672 |
| ING1 | Homo sapiens growth inhibitor p331NG1 (ING1) mRNA, complete cds | Signal, Supressor | AF001954 |
| INS | Homo sapiens insulin (INS), mRNA | Tyrosine Hydroxylase, insulin | NM_000207 |
| IRS4 | Homo sapiens insulin receptor substrate 4 (IRS4) | Insulin | NM_003604 |
| ITGB1 | Integrin, beta 1 (fibronectin receptor, beta polypeptide, antigen CD29 includes MDF2, MSK12); | Signal | X07979 |
| KARP1 | Ku86 autoantigen related protein 1 | Signal | AF039597 |
| KRAS2 | Human K-ras oncogene protein mRNA (KRAS2) | oncogene | M54968 |
| MAP3K7 | Homo sapiens mitogen-activated protein kinase kinase kinase 7 (MAP3K7), mRNA, TAK1 | Signal | NM_003188 |
| MSH6 | Human DNA mismatch repair protein MSH6; mutS alpha 160-kDa subunit; G/T mismatch binding protein (GTMBP; GTBP) | DNArepair | U54777 |
| NR3C1 | Human glucocorticoid receptor alpha mRNA, complete cds | glucocorticoids (Cortisol) | M10901 |
| NRF | Homo sapiens transcription factor NRF | mitochondria & stress | NM_017544 |
| NTE | Homo sapiens mRNA for neuropathy target esterase | esterase | AJ004832 |
| P2Y5 | Homo sapiens purinergic receptor P2Y5 mRNA | Signal | AF000546 |
| PAP | poly(A) polymerase | polymerase | X76770 |
| PGK1 | phosphoglycerate kinase 1 | polymerase | V00572 |
| PIK3C3 | H.sapiens mRNA for phosphatidylinositol 3-kinase, Phosphoinositide-3-kinase, class 3 | Signal | Z46973 |
| PIK3CA | Human phosphoinositide 3'-hydroxykinase p110-alpha subunit mRNA, Phosphoinositide-3-kinase, catalytic, alpha polypeptide | Signal | U79143 |
| POLB | polymerase (DNA directed), beta | polymerase | D29013 |
| POLR2B | polymerase (RNA) II (DNA directed) polypeptide B (140kD) | polymerase | X63563 |
| PPP3CC | calcineurin A catalytic subunit [human, testis, mRNA, 2134 nt]; Protein phosphatase 3 (formerly 2B), catalytic subunit, gamma isoform (calcineurin A gamma) | Signal | S46622 |
| PRKCH | Human protein kinase C-L (PRKCL) mRNA; Protein kinase C, eta | Signal | M55284 |
| PTPN7 | Human mRNA for protein-tyrosine phosphatase; Protein tyrosine phosphatase, non-receptor type 7, HePTP | Signal | D11327 |
| RAB4 | Homo sapiens GTP-binding protein (RAB4) mRNA, complete cds. | oncogene | M28211 |
| RAB7L1 | Homo sapiens mRNA for small GTP-binding protein, complete cds | oncogene | D84488 |
| RAP1A | Human ras-related protein (Krev-1) mRNA, complete cds | Supressor | M22995 |
| RBBP1 | Homo sapiens retinoblastoma-binding protein 1 (RBBP1) mRNA | Signal | NM_002892 |
| RBBP4 | Human chromatin assembly factor 1 p48 subunit (CAF1 p48 subunit); Signal retinoblastoma-binding protein 4 | | X74262 |
| RBBP6 | H.sapiens RBQ-1 mRNA | Signal | X85133 |
| RBBP7 | Human retinoblastoma-binding protein (RbAp46) mRNA, complete cds | Signal | U35143 |
| RPC39 | polymerase (RNA) III (DNA directed) (39kD) | polymerase | U93869 |
| SGK2 | Homo sapiens serum/glucocorticoid regulated kinase 2 | hyperosmotic stress | NM_016276 |
| SLC35A1 | solute carrier family 35 (CMP-sialic acid transporter), member 1 | polymerase | D87969 |
| TAF2F | TATA box binding protein (TBP)-associated factor, RNA polymerase II, F, 55kD | polymerase, TF | U18062 |
| TAF2G | TATA box binding protein (TBP)-associated factor, RNA polymerase II, 32kD | G, polymerase, TF | U21858 |
| TCEB1 | transcription elongation factor B (SIII), polypeptide 1 (15kD, elongin C) | polymerase, TF | L34587 |
| TCEB1L | transcription elongation factor B (SIII), polypeptide 1-like | polymerase, TF | Z47087 |
| TNFRSF6 | H.sapiens mRNA for APO-1 cell surface antigen, FAS | Appoptosis, Cytokine, Signal | X63717 |
| TNFSF10 | Human TNF-related apoptosis inducing ligand TRAIL mRNA, complete cds | Cytokine | U37518 |
| TOP2B | H.sapiens TOP2 mRNA for DNA topoisomerase II (partial).; Topoisomerase topoiosomerase (DNA) II beta (180kD) | | Z15115 |
| TOPBP1 | Homo sapiens mRNA for DNA topoisomerase II binding protein, complete cds | topoiosomerase | AB019397 |

**Table 4:**

| Genes exhibiting significant differences before and after treatment in PB group | | | |
|---|---|---|---|
| Symbol | Name | Category | GenBank ID |
| 5T4 | H.sapiens 5T4 gene for 5T4 Oncofetal antigen | oncogene | Z29083 |
| AANAT | Human serotonin N-acetyltransferase mRNA, complete cds | NAT | U40347 |
| ADCY9 | Homo sapiens adenylate cyclase 9 (ADCY9) | Signal | NM_001116 |
| ADH5 | Human alcohol dehydrogenase class III (ADH5) mRNA | ADH | M29872 |
| ADPRTL1 | ADP-ribosyltransferase (NAD+; poly (ADP-ribose) polymerase)-like 1 | polymerase | AF057160 |
| AKAP6 | Homo sapiens A kinase (PRKA) anchor protein 6 (AKAP6) | Signal | NM_004274 |
| AKR1B1 | Homo sapiens aldo-keto reductase family 1, member B1 (aldose reductase) | hyperosmotic stress | NM_001628 |
| ALDH10 | Human microsomal aldehyde dehydrogenase (ALD10) mRNA | ALDH | U46689 |
| APG-1 | Homo sapiens mRNA for heat shock protein apg-1; Heat shock protein (hsp110 family) | hsp | AB023421 |
| ARNTL | Homo sapiens mRNA for BMAL1a; aryl hydrocarbon receptor nuclear translocator-like | Ah receptor | D89722 |
| ATP2A2 | ATPase, Ca++ transporting, cardiac muscle, slow twitch 2 | ATPase | M23114 |
| ATP5J2 | ATP synthase, H+ transporting, mitochondrial F0 complex, subunit f, isoform 2 | ATPase | AF047436 |
| ATP5JD | ATP synthase, H+ transporting, mitochondrial F1F0, subunit d | ATPase | AF087135 |
| ATP6DV | Vacuolar proton-ATPase, subunit D; V-ATPase, subunit D | ATPase | X71490 |
| ATP6E | ATPase, H+ transporting, lysosomal (vacuolar proton pump) 31kD; Vacuolar proton-ATPase, subunit E; V-ATPase, subunit E | ATPase | X76228 |
| ATP6H | ATPase, H+ transporting, lysosomal (vacuolar proton pump) 9kD | ATPase | Y15286 |
| ATP6S14 | ATPase, vacuolar, 14 kD | ATPase | D49400 |
| BAK1 | Human bcl2 homologous antagonist/killer (BAK) | Appoptosis | U23765 |
| BCL2L1 | H.sapiens bcl-.xL mRNA; BCL2-like 1 | Signal | Z23115 |
| CASP10 | Human apoptotic cysteine protease Mch4 (Mch4) mRNA, complete cds | Appoptosis, Signal | U60519 |
| CCNB2 | Human cyclin B2 mRNA, complete cds | CellCycle | AF002822 |
| CD3E | Human mRNA for T3 epsilon chain (20K) of T-cell receptor (from peripheral blood lymphocytes). | Signal | X03884 |
| CD3G | Human mRNA for T-cell receptor T3 gamma polypeptide, RON alpha | Signal | X04145 |
| CD86 | Human CD86 antigen mRNA, complete cds | Signal | U04343 |
| CDC25C | Human cdc25Hs mRNA, complete cds | CellCycle | M34065 |
| CDC2L5 | Human cdc2-related protein kinase (CHED) mRNA; Cell division cycle 2-like 5 (cholinesterase-related cell division controller) | CellCycle | M80629 |
| CDC37 | Human CDC37 homolog mRNA, complete cds | CellCycle | U63131 |
| CDK7 | H.sapiens CDK activating kinase mRNA | CellCycle | X77743 |
| CDKN2C | Homo sapiens cyclin-dependent kinase inhibitor (CDKN2C) mRNA, complete cds.; p18 | CellCycle | AF041248 |
| CHST1 | Homo sapiens mRNA for keratan sulfate Gal-6-sulfotransferase | sulfotransferase | AB003791 |
| COX4 | Homo sapiens cytochrome c oxidase subunit IV (COX4), nuclear gene encoding mitochondrial protein | mitochondria stress | &NM_001861 |
| COX5A | Homo sapiens cytochrome c oxidase subunit Va | mitochondria & stress | NM_004255 |
| COX6C | Homo sapiens cytochrome c oxidase subunit VIc (COX6C), nuclear gene encoding mitochondrial protein | mitochondria & stress | NM_004374 |
| COX7A2 | Homo sapiens cytochrome c oxidase subunit VIIa polypeptide 2 (liver) (COX7A2), nuclear gene encoding mitochondrial protein | mitochondria stress | & NM_001865 |
| COX7A2L | Homo sapiens cytochrome c oxidase subunit VIIa polypeptide 2 like | mitochondria & stress | NM_004718 |
| COX7B | Homo sapiens cytochrome c oxidase subunit VIIb | mitochondria & stress | NM_001866 |
| COX7C | Homo sapiens cytochrome c oxidase subunit VIIc | mitochondria & stress | NM_001867 |
| CPT1B | Homo sapiens camitine palmitoyltransferase I, muscle (CPT1B) | mitochondria stress | & NM_004377 |
| CSF1R | Human macrophage colony stimulating factor I receptor precursor (CSF1 R); fms proto-oncogene (c-fms) | oncogene | X03663 |
| CSF2RB | Human GM-CSF receptor beta chain mRNA; IL3R-beta | Cytokine, Signal | M59941 |
| CSNK1A1 | Homo sapiens casein kinase I alpha isoform (CSNK1A1) mRNA | Signal | L37042 |
| CYP2A7 | Human cytochrome P450 (CYP2A7) mRNA, complete cds | P450 | U22029 |
| CYP2C19 | Human cytochrome P4502C19 (CYP2C19) mRNA, clone 11a | P450 | M61854 |
| CYP3A5P 1 | Human cytochrome P450 pseudogene mRNA | P450 | L26985 |
| DAXX | Homo sapiens Fas-binding protein Daxx mRNA, complete cds | Signal | AF015956 |
| DCC | Human tumor suppressor protein DCC precursor; colorectal cancersuppressor | Supressor | X76132 |
| DDOST | Human mRNA for KIAA0115 gene; Dolichyl-diphosphooligosac charide-protein glycosyltransferase | UGT | D29643 |
| DOK1 | Docking protein 1, 62kD (downstream of tyrosine kinase 1) | Gap-junciton | U70987 |
| DUSP1 | H.sapiens CL 100 mRNA for protein tyrosine phosphatase, Dual specificity phosphatase 1, MKP1 | Signal | X68277 |
| E2F2 | Homo sapiens transcription factor E2F-2 mRNA, complete cds (clone 9). | TF | L22846 |
| E2F3 | Homo sapiens E2F transcription factor 3(E2F3) | TF | Y10479 |
| EEF1A1 | Homo sapiens eukaryotic translation elongation factor 1 alpha 1 (EEF1A1) | glucocorticoids (Cortisol) | NM_001402 |
| ESD | Homo sapiens esterase D mRNA | esterase | AF112219 |
| FCER1G | Human Fc-epsilon-receptor gamma-chain mRNA; Fc fragment of IgE, high affinity I, receptor for; gamma polypeptide | Signal | M33195 |
| FOS | Homo sapiens v-fos FBJ murine osteosarcoma viral oncogene homolog (FOS), mRNA. | oncogene, Signal, TF | NM_005252 |
| FRAT1 | Homo sapiens frequently rearranged in advanced T-cell lymphomas (FRAT1) mRNA | Signal | NM_005479 |
| G22P1 | Human Ku protein subunit mRNA; Thyroid autoantigen 70kD (Ku antigen) | Signal | M32865 |
| GJA5 | gap junction protein, alpha 5, 40kD (connexin 40) | Gap-junciton | L34954 |
| GNA15 | Human G-alpha 16 protein mRNA, complete cds; Guanine nucleotide binding protein (G protein), alpha 15 (Gq class) | Signal | M63904 |
| GNB3 | Human guanine nucleotide-binding protein beta-3 subunit mRNA; Guanine nucleotide binding protein (G protein), beta polypeptide 3 | Signal | M31328 |
| HLA-DRA | Human HLA-DR alpha-chain mRNA; Class II MHC alpha | Signal | K01171 |
| HLA-DRB 1 | Human mRNA for HLA class II DR-beta 1 (Dw14); Class II MHC beta | Signal | X02902 |
| HMG1 | Human mRNA for high mobility group-1 protein (HMG-1). | sulfotransferase | X12597 |
| HSBP1 | Homo sapiens heat shock factor binding protein 1 HSBP1 mRNA; Heat shock factor binding protein 1 | hsp | AF068754 |
| HSPA4 | Human heat shock protein 70 (hsp70) mRNA; Heat shock 70kD protein 4 | hsp | L12723 |
| HSPCB | Human 90-kDa heat-shock protein gene, cDNA; Heat shock 90kD protein 1, beta | hsp | M16660 |
| HSPD1 | Heat shock 60kD protein 1 (chaperonin) | hsp | M34664 |
| HSPE1 | Human chaperonin 10 mRNA; Heat shock 10kD protein 1 | hsp | U07550 |
| IGF1R | Human mRNA for insulin-like growth factor I receptor | GF, Signal | X04434 |
| IGFBP7 | prostacyclin-stimulating factor [human, cultured diploid fibroblastcells mRNA, 1124 nt]. | GF | S75725 |
| IL1R2 | H.sapiens IL-1R2 mRNA for type II interleukin-1 receptor, (cell line CB23). | Cytokine | X59770 |
| IL2RG | Human mRNA for interleukin 2 receptor gamma chain | Cytokine, Signal | D11086 |
| ITGB2 | Human leukocyte adhesion protein (LFA-1/Mac-1/p150,95 family) beta subunit mRNA, CD18 | Signal | M15395 |
| LOC51189 | ATPase inhibitor precursor | ATPase | AB029042 |
| MADD | Homo sapiens MAP kinase-activating death domain protein (MADD) mRNA | Signal | U77352 |
| MAFG | Homo sapiens basic-leucine zipper transcription factor MafG (MAFG), mRNA, complete cds | oncogene, TF | AF059195 |
| MAX | H.sapiens max mRNA | Signal | X60287 |
| NFATC1 | Human NF-ATc mRNA, complete cds | Signal, TF | U08015 |
| NFATC3 | Homo sapiens NF-AT4c mRNA, complete cds | Signal, TF | L41067 |
| NME2 | Human putative NDP kinase (nm23-H2S) mRNA, complete cds; c-myc purine-binding transcription factor puf | TF | M36981 |
| NR1H4 | Human famesol receptor HRR-1 (HRR-1) mRNA, complete cds | NR1(FXR) | U68233 |
| NRF | Homo sapiens transcription factor NRF | mitochondria & stress | NM_017544 |
| NTRK1 | Human mRNA of transforming tyrosine kinase protein trk oncogene; high-affinity nerve growth factor receptor precursor; | oncogene | X03541 |
| PDAP1 | Human PDGF associated protein mRNA (PAP) | GF | U41745 |
| PDCD8 | Homo sapiens apoptosis-inducing factor AIF mRNA, nuclear gene encodingmitochondrial protein; Programmed cell death 8 | Signal | AF100928 |
| PGK1 | phosphoglycerate kinase 1 | polymerase | V00572 |
| PIK3C3 | H.sapiens mRNA for phosphatidylinositol 3-kinase, Phosphoinositide-3-kinase, class 3 | Signal | Z46973 |
| PLCB4 | Homo sapiens phospholipase C beta 4 (PLCB4) mRNA; Phospholipase C, beta 4 | Signal | L41349 |
| POLR2B | polymerase (RNA) II (DNA directed) polypeptide B (140kD) | polymerase | X63563 |
| POLRMT | polymerase (RNA) mitochondrial (DNA directed) | polymerase | U75370 |
| POU2F1 | Human mRNA for octamer-binding protein Oct-1; POU domain, class 2, transcription factor 1 | TF | X13403 |
| POU2F2 | Human lymphoid-specific transcription factor mRNA; POU domain, class 2, transcription factor 2 | TF | M36542 |
| PPARA | Human peroxisome proliferator activated receptor mRNA, complete cds | PPAR | L02932 |
| PPARD | Human peroxisome proliferator activated receptor mRNA, complete cds | PPAR | L07592 |
| PRKCBP1 | Homo sapiens protein kinase C-binding protein RACK7 mRNA, partial cds; Protein kinase C binding protein 1 | Signal | U48251 |
| PRKCH | Human protein kinase C-L (PRKCL) mRNA; Protein kinase C, eta | Signal | M55284 |
| PRKCQ | Human protein kinase C theta (PKC) mRNA; Protein kinase C, theta | Signal | L07032 |
| PSMC1 | Proteasome (prosome, macropain) 26S subunit, ATPase, 1 | ATPase | L02426 |
| PTPN11 | Homo sapiens SH-PTP3 mRNA for protein-tyrosine phosphatase; Protein tyrosine phosphatase, non-receptor type 11; Shp2 | Signal | D13540 |
| PTPN6 | H.sapiens PTP1C mRNA for protein-tyrosine phosphatase 1C.; Protein tyrosine phosphatase, non-receptor type 6; SHP-1 | Signal | X62055 |
| PTPN7 | Human mRNA for protein-tyrosine phosphatase; Protein tyrosine | Signal | D11327 |
| | phosphatase, non-receptor type 7, HePTP | | |
| RAB7L1 | Homo sapiens mRNA for small GTP-binding protein, complete cds | oncogene | D84488 |
| RASSF1 | Homo sapiens putative tumor suppressor protein (RDA32) mRNA, complete cds | Supressor | AF061836 |
| RBBP2 | RBP2=retinoblastoma binding protein 2 [human, Nalm-6 pre-B cell leukemia, mRNA, 6455 nt]. | Signal | S66431 |
| RDS | Retinal degeneration, slow (retinitis pigmentosa 7) | ATPase | M73531 |
| RPA40 | RNA polymerase I subunit | polymerase | AF008442 |
| RXRG | Human retinoid X receptor-gamma mRNA, complete cds | RXR | U38480 |
| SGK2 | Homo sapiens serum/glucocorticoid regulated kinase 2 | hyperosmotic stress | NM_016276 |
| SLC35A1 | solute carrier family 35 (CMP-sialic acid transporter), member 1 | polymerase | D87969 |
| SLC7A2 | Homo sapiens solute carrier family 7 (cationic amino acid transporter, system), member 2 | y+ hyperosmotic stress | NM_003046 |
| ST14 | Human SNC19 mRNA sequence Suppression of tumorigenicity 14 (colon carcinoma, matriptase, epithin) | Supressor | U20428 |
| STAT3 | Homo sapiens DNA-binding protein (APRF) mRNA, complete cds | Signal, TF | L29277 |
| STAT5 | Homo sapiens signal transducer and activator of transcription (STAT5) mRNA | Signal, TF | L41142 |
| STAT5B | Human signal transducer and activator of transcription Stat5B mRNA, complete cds | TF | U47686 |
| STAT6 | Human transcription factor IL-4 Stat mRNA, complete cds | Signal, TF | U16031 |
| STIP1 | Homo sapiens stress-induced-phosphoprotein 1 (Hsp70/Hsp90-or ganizing protein) | stress | NM_006819 |
| TAF2F | TATA box binding protein (TBP)-associated factor, RNA polymerase II, F, 55kD | polymerase, TF | U18062 |
| TCEB1 | transcription elongation factor B (SIII), polypeptide 1 (15kD, elongin C) | polymerase, TF | L34587 |
| TCEB1L | transcription elongation factor B (SIII), polypeptide 1-like | polymerase, TF | Z47087 |
| TCF15 | Human basic helix-loop-helix transcription factor mRNA, complete cds | Signal, TF | U08336 |
| TCF3 | Human transcription factor (E2A) mRNA, complete cds | Signal, TF | M31523 |
| TCF7L2 | Homo sapiens mRNA for hTCF-4 | Signal, TF | Y11306 |
| TCFL1 | Human YL-1 mRNA for YL-1 protein (nuclear protein with DNA-binding ability), complete cds | Signal, TF | D43642 |
| TFDP2 | Human DP2 (Humdp2) mRNA; Transcription factor Dp-2 (E2F dimerization TF partner 2) | | U18422 |
| TGFB1 | Human transforming growth factor-beta (TGF-beta; TGFB) | GF, Signal | X02812 |
| TPST2 | Homo sapiens tyrosylprotein sulfotransferase-2 mRNA | sulfotransferase | AF049891 |
| TRA@ | Human mRNA for T-cell receptor alpha chain (TCR-alpha). | Signal | X02592 |
| TSSC1 | Homo sapiens tumor suppressing STF cDNA 1 (TSSC1) mRNA, complete cds | Supressor | AF019952 |
| VAV1 | Human mRNA for vav oncogene | oncogene, Signal | X16316 |
| WISP2 | Homo sapiens connective tissue growth factor related protein WISP-2 (WISP2) mRNA, complete cds. | Signal | AF100780 |

**Table 5:**

| Changes in Hamilton scores before and after treatment | | |
|---|---|---|
| | Before treatment | After treatment |
| #02 | 20 | 4 |
| #04 | 26 | 25 |
| #05 | 25 | 9 |
| #06 | 19 | 10 |
| #07 | 12 | 2 |
| #10 | 16 | 3 |
| #13 | 29 | 7 |
| #14 | 19 | 5 |
| #15 | 31 | 9 |
| #16 | 27 | - |
| #17 | 19 | 3 |
| #29 | 28 | 8 |
| #30 | 34 | 7 |
| #31 | 15 | 3 |
| #33 | 23 | 2 |

| | | |
|---|---|---|
| "-": no data | | |

### [Example 2] Diagnosis of depression using diagnostic marker

The samples obtained from patients afflicted with depression and the samples obtained from healthy volunteers were employed to cluster the patients afflicted with depression and the healthy volunteers and to evaluate the course of treatment for the patients afflicted with depression.

### 1. Subjects

Three patients afflicted with depression and three healthy volunteers were employed as the subjects. Diagnosis was made in accordance with a depressive episode specified in the International Classification of Diseases, 10th revision (ICD-10). Patients with serious physical complications or those taking therapeutic agents for physical diseases were excluded. The samples obtained from 6 subjects were concealed whether they were patients afflicted with depression or healthy volunteers. Those samples were designated as Subjects A, B, C, D, E, and F.

### 2. Analysis of gene expression

Blood (5 ml) was collected from the subjects, and total RNA was extracted using a PAXgene Blood RNA System (Qiagen). The yield of total RNA was 5 µg to 15 µg. Subsequently, 5 µg of total RNA extracted from each subject was separated, annealed with an oligo (dT) 24 primer comprising a T7 promoter sequence added thereto, and first-strand DNA was synthesized. Thereafter, this first-strand DNA was used as a template to synthesize second-strand DNA having a T7 promoter sequence. Finally, the second-strand DNA was used as a template to synthesize RNA with the aid of T7 RNA polymerase. A random hexamer was annealed to 6 µg of RNA to conduct a reverse transcriptase reaction, and Cy5-dCTP was incorporated into the strand. Thus, fluorescence-labeled cDNA was synthesized.

For comparison, blood was collected from healthy volunteers having the same age and sex conditions with the subjects, and Cy3-cDNA was synthesized in the same manner as in the case of the patients' samples. Cy5-cDNA prepared from each subject's sample (6 µg) was mixed with the equivalent amount of Cy3-cDNA as a standard sample, the resultant was applied to a DNA chip (a DNA chip for analyzing drug response, Hitachi Co., Ltd.), and hybridization was carried out at 62°C for 12 hours. After washing, fluorescence intensity at each spot was assayed using a scanner (ScanArray 5000, GSI-Lumonics), and the differences in the expression intensities of each gene between the standard sample and the sample obtained from the subject were determined using quantifying software (QuantArray, GSI-Lumonics).

### 3. Classification of subjects

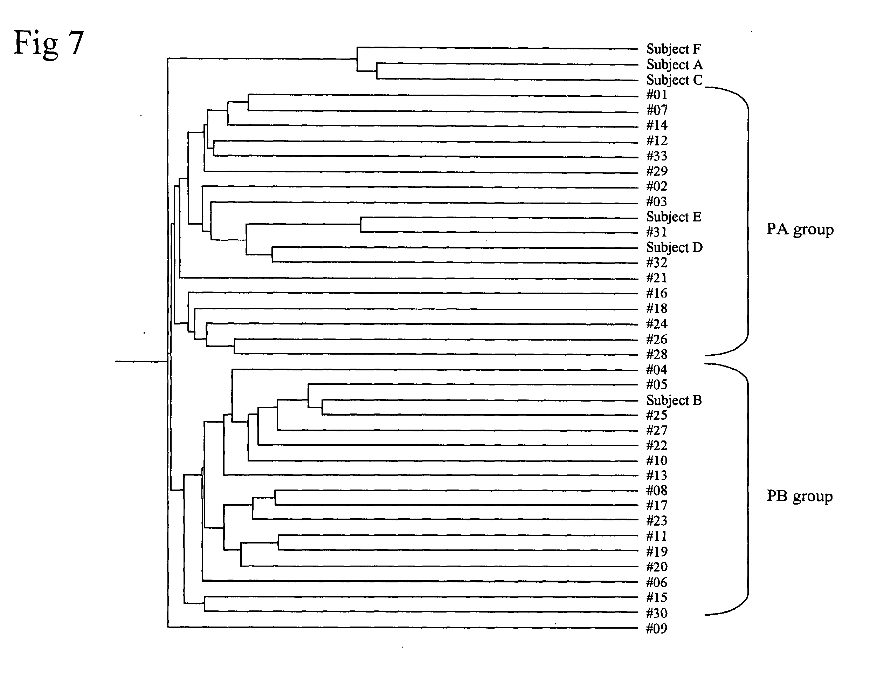

In accordance with the method described in Example 1, these 6 subjects were subjected to hierarchical clustering based on the cosine coefficient distance without a weight between clusters with the 33 subjects for patient/healthy volunteer comparison who had been already analyzed. This analysis demonstrated that Subjects D and E belonged to the PA group, Subject B belonged to the PB group, and Subjects A, C, and F did not belong to either group (Fig. 7). The concealed sample names were examined in relation to the results of clustering. This demonstrated that Subjects B, D, and E were patients afflicted with depression, and Subjects A, C, and F were healthy volunteers, which were completely consistent with the results of clustering.

### 4. Evaluation of course of treatment in accordance with type

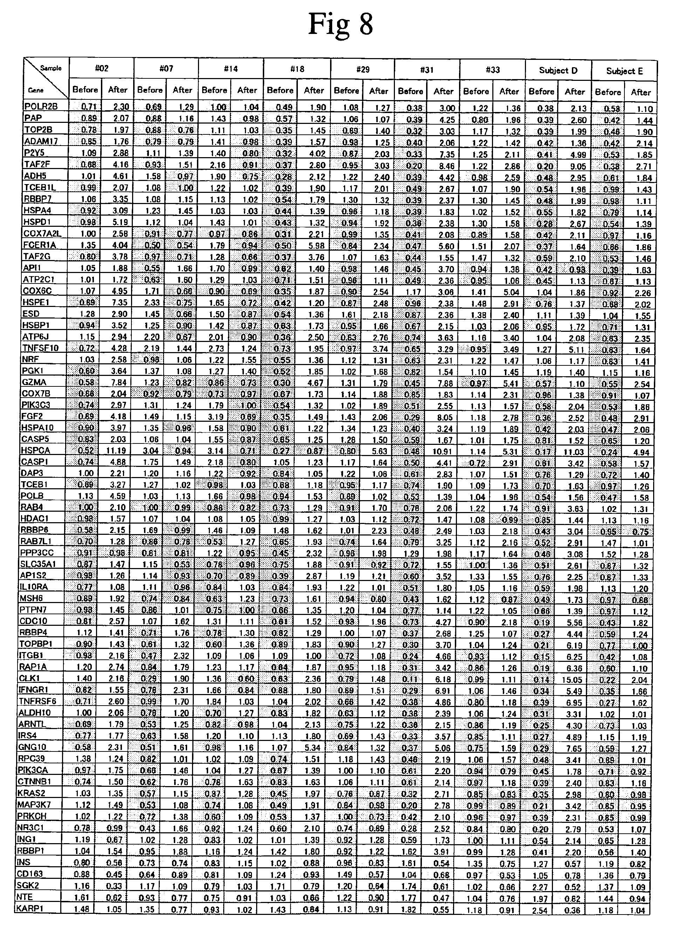

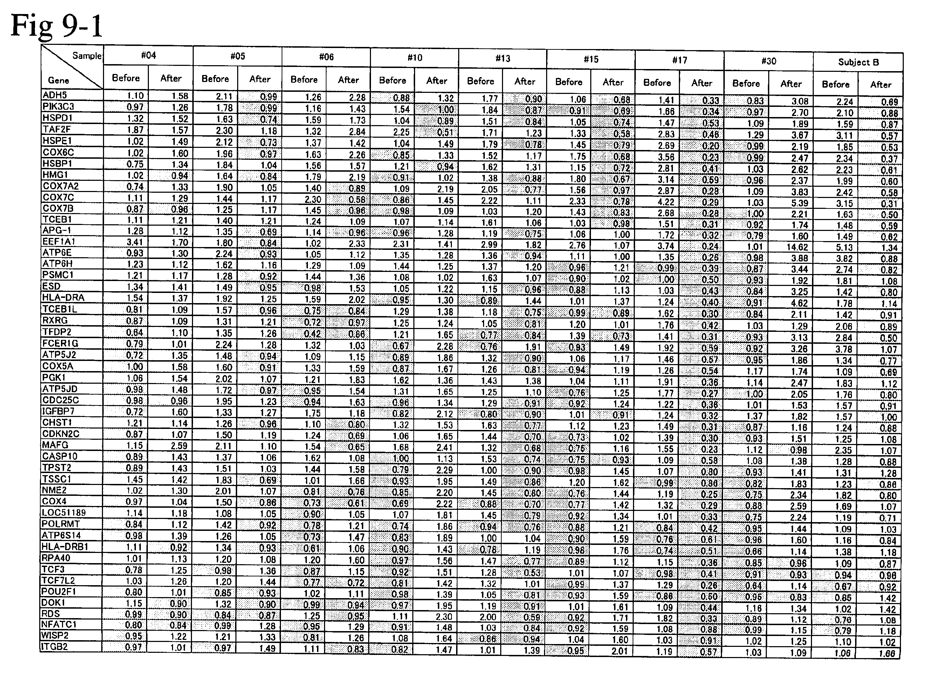

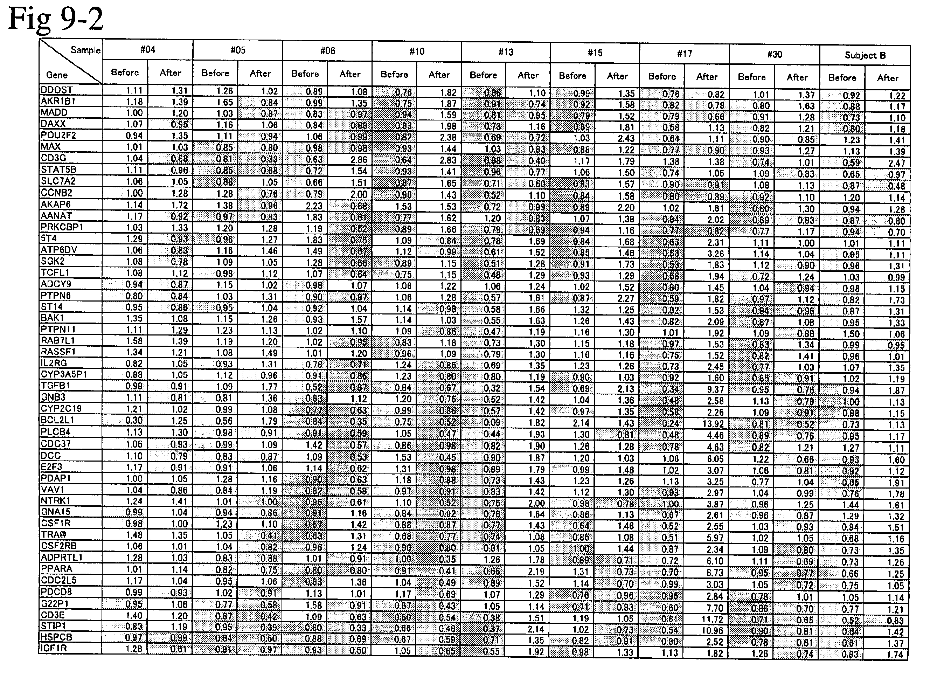

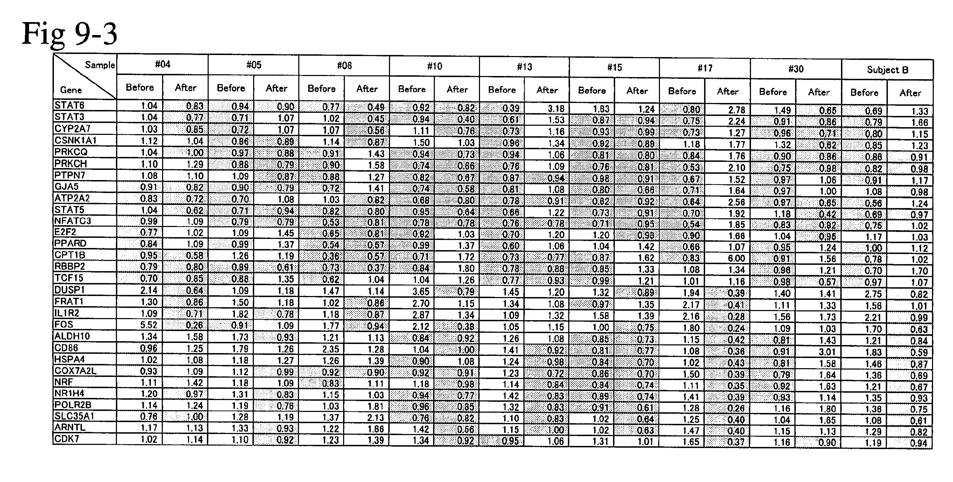

Subsequently, the samples obtained from Subjects B, D, and E after treatment involving the use of antidepressants and the samples thereof before treatment were similarly subjected to analysis via DNA chips. The groups of genes listed in Table 3 were employed to observe changes in the gene expression patterns before and after treatment for Subjects D and E of the PA group. Similarly, the groups of genes listed in Table 4 were employed for Subject B of the PB group. After treatment, the gene expression patterns of all the patients were reversed from those before treatment. This indicates that the clinical conditions are in recovery trends (Fig. 8, Fig. 9).

### 5. Examination (comparison with Hamilton scaling)

The Hamilton scores of 3 patients afflicted with depression were as follows: Subject B: 22 points before treatment and 6 points after treatment; Subject D: 15 points before treatment and 1 point after treatment; and Subject E: 30 points before treatment and 2 points after treatment. Thus, the Hamilton scores were extremely consistent with the recovery trends of the clinical conditions indicated by the expression patterns of the groups of genes. Changes in the Hamilton scores before and after treatment are shown in Table 6.

**Table 6:**

| Changes in Hamilton scores before and after treatment | | |
|---|---|---|
| | Before treatment | After treatment |
| Subject B | 30 | 2 |
| Subject D | 22 | 6 |
| Subject E | 15 | 1 |

### 6. Conclusion

As is apparent from the foregoing, diagnosis of depression via analysis of expression levels of a specific group of genes was extremely consistent with the results attained by clinical finding in terms of classification and evaluation of the course of treatment of patients afflicted with depression. This indicates that the present invention is very effective.

### [Example 3] Selection of diagnostic marker

### 1. Patients and healthy volunteers

Target patients were those who had agreed with the written description for participating in the research for developing the present diagnostic method selected from among untreated patients afflicted with depression who had visited the Department of Psychiatry and Neurology of the Tokushima University Hospital between November 2001 and February 2004. This research was approved by the ethics committee of Tokushima University Hospital. Diagnosis was made in accordance with a depressive episode specified in the International Classification of Diseases, 10th revision (ICD-10). Patients with serious physical complications or those taking therapeutic agents for physical diseases were excluded. Healthy volunteers with the same sex and age conditions with each patient were selected for comparison.

Thirty two patients whose samples before treatment had been obtained were 20 males and 12 females aged 23 to 74 (45.1 years old on average), and their Hamilton scores were between 10 and 35 points (21.3 points on average).

Samples were obtained from 16 patients after the treatment. They were 9 males and 7 females aged 23 to 70 (47.5 years old on average), and their Hamilton scores were between 1 and 10 (4.3 points on average). Treatment was mainly carried out by medication using antidepressants. The remission of symptoms was determined based on general clinical diagnosis. After treatment, all the samples satisfied the standard of having scores of 7 or less on the Hamilton Rating Scale, which are generally regarded as representing remission of symptoms, or the standard such that the Hamilton scores were reduced to half or less those before treatment. Thus, all the samples were determined to have reached the state of remission after treatment.

### 2. Analysis of gene expression

Blood (5 ml) was collected from the patients, and total RNA was extracted using a PAXgene Blood RNA System (Qiagen). Blood was collected by a doctor or nurse between 10:00 am and 1:00 pm from the patients under fasting conditions through cubitus veins under resting conditions. The yield of total RNA was 5 µg to 15 µg.

Subsequently, 5 µg of total RNA extracted from each patient was separated, annealed with an oligo (dT) 24 primer comprising a T7 promoter sequence added thereto, and first-strand DNA was synthesized. Thereafter, this first-strand DNA was used as a template to synthesize second-strand DNA having a T7 promoter sequence. Finally, the second-strand DNA was used as a template to synthesize RNA with the aid of T7 RNA polymerase. A random hexamer was annealed to 6 µg of the synthesized RNA to conduct a reverse transcriptase reaction, and Cy5-dCTP was incorporated into the strand. Thus, fluorescence-labeled cDNA was synthesized.

In a manner similar to the case of the patients, 5 ml of blood was collected from each of 32 healthy volunteers having the same sex and age conditions with the patients, and total RNA was then extracted. cDNA was similarly synthesized except for the use of Cy3 as a fluorescent label.

When comparing samples of a single subject before and after treatment, cDNA labeled with Cy3 and cDNA labeled with Cy5 were synthesized from the samples before and after treatment, respectively.

Equivalent amounts of two types of cDNAs for comparison and analysis were mixed, the resultant was applied to a DNA chip (Stress Chip, Hitachi Co., Ltd.), and hybridization was carried out at 62°C for 12 hours. After washing, fluorescence intensity at each spot was assayed using a scanner (ScanArray 5000, GSI-Lumonics). Differences in gene expression levels between samples obtained from patients and samples obtained from healthy volunteers or those between samples obtained from a single patient before and after treatment were determined.

### 3. Data analysis

### (1) Selection of marker gene for depression

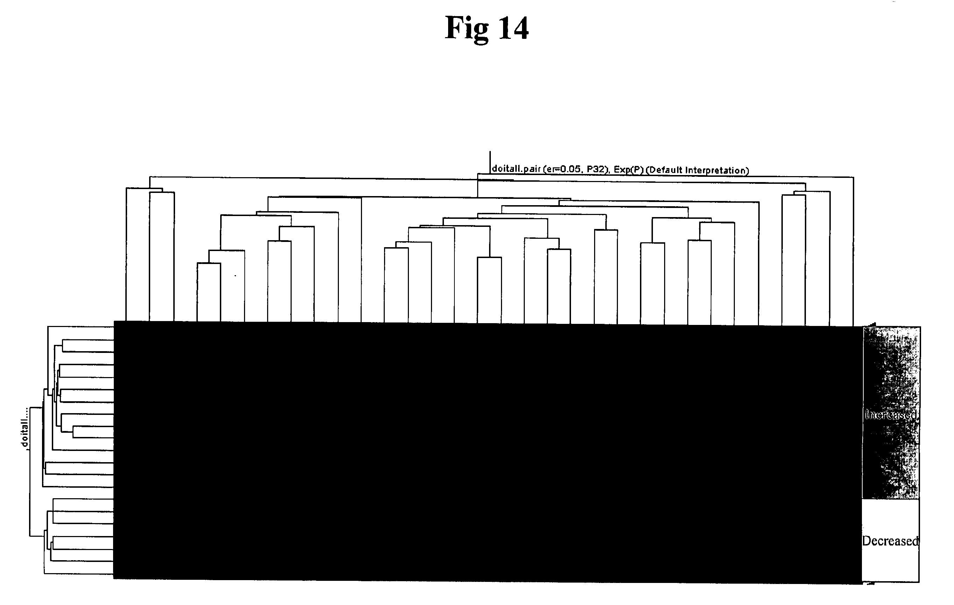

A group of genes (801 genes) having fluorescence intensities of 300 or higher for Cy5 or Cy3 in all 48 groups of data was selected as the object of analysis. Among the data on patient/healthy volunteer comparison, the gene with a significantly higher or lower expression level was selected via a significant difference test. There were 14 genes of the patient with a significantly higher expression level compared to that of the healthy volunteer and 7 genes thereof with a significantly lower expression level (Fig. 14, Table 7). These 21 genes are useful for evaluating whether or not the subject has been afflicted with depression, i.e., they are useful as marker genes for depression. Among them, the expression levels of HLA-G, HRH4, PSMB9, ATP2A2, SCYA5, SLC6A4, CASP6, CSF2, HSD3B1, and RAB9 were significantly varied, and thus, they were considered to be particularly useful marker genes for depression.

**Table 7:**

| Group of genes exhibiting significant differences between patient and healthy volunteer | | | |
|---|---|---|---|
| Symbol | Name | Category | GenBank ID |
| HLA-G | HLA-G histocompatibility antigen, class I, G | - | M32800 |
| HRH4 | histamine H4 receptor | - | NM_021624 |
| PSMB9 | proteasome (prosome, macropain) subunit, beta type, 9 (large multifunctional protease 2) | - | BC008795 |
| ATP2A2 | ATPase, Ca++ transporting, cardiac muscle, slow twitch 2 | ATPase | M23114 |
| SCYA5 | Human T cell-specific protein (RANTES) mRNA, Small inducible cytokine A5 | Cytokine | M21121 |
| SLC6A4 | solute carrier family 6 (neurotransmitter transporter, serotonin), member 4 | - | NM_001045 |
| CASP6 | Human cysteine protease Mch2 isoform alpha (Mch2) mRNA, complete cds | Appoptosis, Signal | U20536 |
| CSF2 | Human T-cell granulocyte-macrophage colony stimulating factor (GM-CSF) mRNA | Cytokine, Signal | M10663 |
| HSD3B1 | Homo sapiens hydroxy-delta-5-steroid dehydrogenase, 3 beta- and steroid delta-isomerase 1 (HSD3B1) | glucocorticoids (Cortisol) | NM_000862 |
| RAB9 | Human small GTP binding protein Rab9 mRNA, complete cds. | oncogene | U44103 |
| TPR | H.sapiens tpr mRNA; Translocated promoter region (to activated MET oncogene) | oncogene | X66397 |
| ABCF1 | Homo sapiens TNF-alpha stimulated ABC protein (ABC50) mRNA, complete cds | ABC transporter | AF027302 |
| AKAP6 | Homo sapiens A kinase (PRKA) anchor protein 6 (AKAP6) | Signal | NM_004274 |
| PSMC5 | Proteasome (prosome, macropain) 26S subunit, ATPase, 5 | ATPase | AF035309 |
| Hs.14438 | Homo sapiens, Similar to histamine N-methyltransferase, clone MGC:14500 IMAGE:4249496, mRNA, complete cds | - | BC005907 |
| KLK6 | kallikrein 6 (neurosin, zyme) | - | AF013988 |
| STIP1 | Homo sapiens stress-induced-phosphoprotein 1 (Hsp70/Hsp90-organizing protein) | stress | NM_006819 |
| PGK1 | phosphoglycerate kinase 1 | polymerase | V00572 |
| PSMD5 | proteasome (prosome, macropain) 26S subunit, non-ATPase, 5 | - | D31889 |
| TGFBR3 | Human transforming growth factor-beta type III receptor (TGF-beta) mRNA, complete cds | GF | L07594 |
| TSSC1 | Homo sapiens tumor suppressing STF cDNA 1 (TSSC1) mRNA, complete cds | Supressor | AF019952 |

### (2) Selection of marker gene for classification

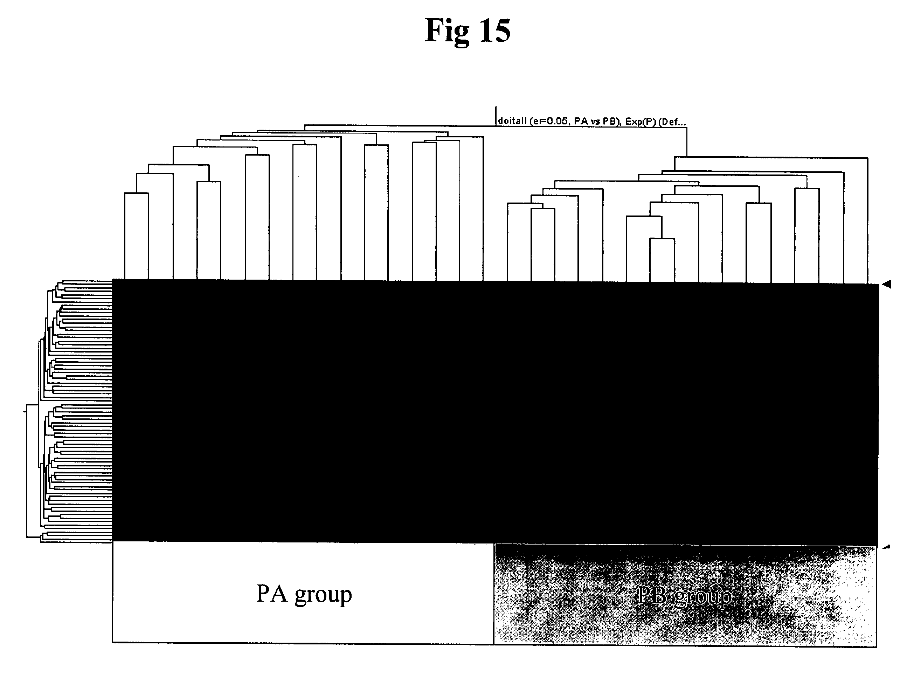

Thirty two pairs of subjects for patient/healthy volunteer comparison were subjected to cluster analysis utilizing all the genes (801 genes). Analysis was carried out by hierarchical clustering based on the cosine coefficient distance without a weight between clusters. This cluster analysis demonstrated that the patient/healthy volunteer comparison samples were roughly divided into 2 groups. Such 2 groups were designated as the PA group and the PB group. The 32 pairs of subjects for patient/healthy volunteer comparison were divided into the PA group (16 pairs) and the PB group (16 pairs). In order to extract the genes that were peculiar to the PA group and to the PB group, these groups were compared to each other. There were 75 genes that exhibited significant differences between the PA group and the PB group (Fig. 15, Table 8). These 75 genes are useful for assigning patients afflicted with depression to the PA or PB group, i.e., they are useful as marker genes for classification the patients afflicted with depression. Among them, the expression levels of HSPE1, PSMA4, ADH5, PSMA6, COX17, HMG1, GPR24, COX6C, FGF2, and COX7C were significantly varied, and thus, they were considered to be particularly useful marker genes for classification.

**Table 8:**

| Group of genes exhibiting significant differences between PA group and PB group | | | |
|---|---|---|---|
| Symbol | Name | Category | GenBank ID |
| HSPE1 | Human chaperonin 10 mRNA; Heat shock 10kD protein 1 | hsp | U07550 |
| PSMA4 | proteasome (prosome, macropain) subunit, alpha type, 4 | - | BC005361 |
| ADH5 | Human alcohol dehydrogenase class III (ADH5) mRNA | ADH | M29872 |
| PSMA6 | proteasome (prosome, macropain) subunit, alpha type 6 | - | X59417 |
| COX17 | Homo sapiens COX17 (yeast) homolog, cytochrome c oxidase assembly protein | mitcondria & stress | NM_005694 |
| HMG1 | Human mRNA for high mobility group-1 protein (HMG-1). | sulfotransferase | X12597 |
| GPR24 | G protein-coupled receptor 24 | - | BC001736 |
| COX6C | Homo sapiens cytochrome c oxidase subunit Vic (COX6C), nuclear gene encoding mitochondrial protein | mitcondria & stress | NM_004374 |
| FGF2 | Human basic fibroblast growth factor (FGF) mRNA (BFGF; FGFB; FGF2) | GF | M27968 |
| COX7C | Homo sapiens cytochrome c oxidase subunit VIIc | mitcondria & stress | NM_001867 |
| CCNA2 | Human mRNA for cyclin A; Cyclin A2 | CellCycle | X51688 |
| PTGER3 | prostaglandin E receptor 3 (subtype EP3) | - | X83860 |
| APG-1 | Homo sapiens mRNA for heat shock protein apg-1; Heat shock protein (hsp110 family) | hsp | AB023421 |
| HSPCA | Homo sapiens Hsp89-alpha-delta-N mRNA; Heat shock 90kD protein 1, alpha | hsp | AF028832 |
| UBL1 | ubiquitin-like 1 (sentrin) | Gap-junciton | U61397 |
| UCHL3 | Human ubiquitin carboxyl-terminal hydrolase (PGP 9.5, UCH-L3) isozyme L3 mRNA | esterase | M30496 |
| HINT | Homo sapiens protein kinase C inhibitor (PKCI-1) mRNA, Histidine triad nucleotide-binding protein | Signal | U51004 |
| BDKRB2 | Homo sapiens bradykinin receptor B2 | heart stress | NM_000623 |
| SOD1 | Homo sapiens superoxide dismutase 1, soluble (amyotrophic lateral sclerosis 1 (adult)) (SOD1); Superoxide dismutase 1, soluble (amyotrophic lateral sclerosis 1 (adult)) | SOD | NM_000454 |
| IL13RA2 | Human interleukin-13 receptor mRNA, complete cds | Cytokine | U70981 |
| HSBP1 | Homo sapiens heat shock factor binding protein 1 HSBP1 mRNA; Heat shock factor binding protein 1 | hsp | AF068754 |
| EEF1A1 | Homo sapiens eukaryotic translation elongation factor 1 alpha 1 (EEF1A1) | glucocorticoids (Cortisol) | NM_001402 |
| PSMA7 | proteasome (prosome, macropain) subunit, alpha type, 7 | - | BC004427 |
| PSMA3 | proteasome (prosome, macropain) subunit, alpha type, 3 | - | BC005265 |
| UFD1L | Ubiquitin fusion degradation 1-like | - | BC005087 |
| CCNH | Human cyclin H mRNA, complete cds | CellCycle | U11791 |
| ATP6J | ATPase, H+ transporting, lysosomal (vacuolar proton pump), member J | ATPase | AF038954 |
| HGF | Human hepatocyte growth factor mRNA (HGF); scatter factor (SF); hepatopoeitin A | GF | M60718 |
| PRDX4 | peroxiredoxin 4 | - | BC003609 |
| GZMA | Human Hanukah factor serine protease (HuHF) mRNA (cytotoxic T-lymphocyte-associated serine esterase 3) | esterase | M18737 |
| PSMD10 | proteasome (prosome, macropain) 26S subunit, non-ATPase, 10 | - | NM_002814 |
| COX7A2 | Homo sapiens cytochrome c oxidase subunit VIIa polypeptide 2 (liver) (COX7A2), nuclear gene encoding mitochondrial protein | mitcondria & stress | NM_001865 |
| HSJ2 | Human heat shock protein, E. coli DnaJ homologue mRNA, complete cds; Heat shock protein, DNAJ-like 2 | hsp | L08069 |
| B2M | beta-2-microglobulin | - | AY007153 |
| TCEB1 | transcription elongation factor B (SIII), polypeptide 1 (15kD, elongin C) | polymerase, TF | L34587 |
| HTR6 | 5-hydroxytryptamine (serotonin) receptor 6 | - | NM_000871 |
| TXN | thioredoxin | - | X77584 |
| HSPD1 | Heat shock 60kD protein 1 (chaperonin) | hsp | M34664 |
| PSMC6 | Proteasome (prosome, macropain) 26S subunit, ATPase, 6 | ATPase | AF006305 |
| POLR2A | polymerase (RNA) II (DNA directed) polypeptide A (220kD); H.sapiens mRNA for RNA polymerase II largest subunit | polymerase | X63564 |
| HSPA4 | Human heat shock protein 70 (hsp70) mRNA; Heat shock 70kD protein 4 | hsp | L12723 |
| DAP3 | Human ionizing radiation resistance conferring protein mRNA; Death associated protein 3 | Appoptosis | U18321 |
| NME2 | Human putative NDP kinase (nm23-H2S) mRNA, complete cds; c-myc purine-binding transcription factor puf | TF | M36981 |
| CD86 | Human CD86 antigen mRNA, complete cds | Signal | U04343 |
| IGBP1 | Immunoglobulin (CD79A) binding protein 1 | Signal | Y08915 |
| WISP3 | Homo sapiens connective tissue growth factor related protein WISP-3 (WISP3) mRNA, complete cds. | Signal | AF100781 |
| COPS5 | Human Jun activation domain binding protein mRNA, complete cds | oncogene | U65928 |
| DBI | diazepam binding inhibitor (GABA receptor modulator, acyl-Coenzyme A binding protein) | - | BC006466 |
| SCYA7 | Homo sapiens mRNA for monocyte chemotactic protein-3 (MCP-3), Small inducible cytokine A7 (monocyte chemotactic protein 3) | Cytokine | X72308 |
| NCOR2 | Human silencing mediator of retinoid and thyroid hormone action (SMRT) mRNA, Nuclear receptor co-repressor 2 | NR | U37146 |
| PSMB1 | proteasome (prosome, macropain) subunit, beta type, 1 | - | BC000508 |
| DMBT1 | Homo sapiens mRNA for DMBT1 6 kb transcript variant 1 (DMBT1/6kb.1). | Supressor | AJ000342 |
| POLR2H | Human RNA polymerase II subunit (hsRPB8) mRNA; polymerase (RNA) (DNA directed) polypeptide H | II polymerase | U37689 |
| PSMA1 | proteasome (prosome, macropain) subunit, alpha type, 1 | - | BC002577 |
| PAP | poly(A) polymerase | polymerase | X76770 |
| HSPA10 | Homo sapiens heat shock 70kD protein 10 (HSC71) (HSPA10), mRNA | hsp | NM_006597 |
| PSMA5 | proteasome (prosome, macropain) subunit, alpha type, 5 | - | X61970 |
| P2Y5 | Homo sapiens purinergic receptor P2Y5 mRNA | Signal | AF000546 |
| SLC35A1 | solute carrier family 35 (CMP-sialic acid transporter), member 1 | polymerase | D87969 |
| COX7B | Homo sapiens cytochrome c oxidase subunit VIIb | mitcondria & stress | NM_001866 |
| HTR2A | 5-hydroxytryptamine (serotonin) receptor 2A | - | X57830 |
| KLK12 | Homo sapiens kallikrein 12 (KLK12), mRNA | - | NM_019598 |
| Hs.35129 0 | Homo sapiens cDNA FLJ30648 fis, clone CTONG2006449, moderately similar to Drosophila melanogaster 26S proteasome regulatory complex subunit p42A mRNA | - | AK055210 |
| ACE | Homo sapiens dipeptidyl carboxypeptidase 1 (angiotensin I converting enzyme) (ACE) | angiotensin | NM_000789 |
| NR1H4 | Human famesol receptor HRR-1 (HRR-1) mRNA, complete cds | NR1(FXR) | U68233 |
| KIAA010 7 | KIAA0107 gene product | - | BC000904 |
| COX7A2 L | Homo sapiens cytochrome c oxidase subunit VIIa polypeptide 2 like | mitcondria & stress | NM_004718 |
| VCP | valosin-containing protein | - | BC007562 |
| RPA40 | RNA polymerase I subunit | polymerase | AF008442 |
| TXNL | thioredoxin-like, 32kD | - | BC001156 |
| TAF2G | TATA box binding protein (TBP)-associated factor, RNA polymerase II, G, 32kD | polymerase, TF | U21858 |
| TGFBR1 | Human activin receptor-like kinase (ALK-5) mRNA, complete cds | GF, Signal | L11695 |
| DIA4 | Human, NAD(P)H:menadione oxidoreductase mRNA | NQO | J03934 |
| MAP2K3 | Human mRNA for MAP kinase kinase 3b complete cds, MEK3 | Signal | D87116 |
| ATP5JD | ATP synthase, H+ transporting, mitochondrial F1F0, subunit d | ATPase | AF087135 |

### (3) Selection of diagnostic marker gene for each group

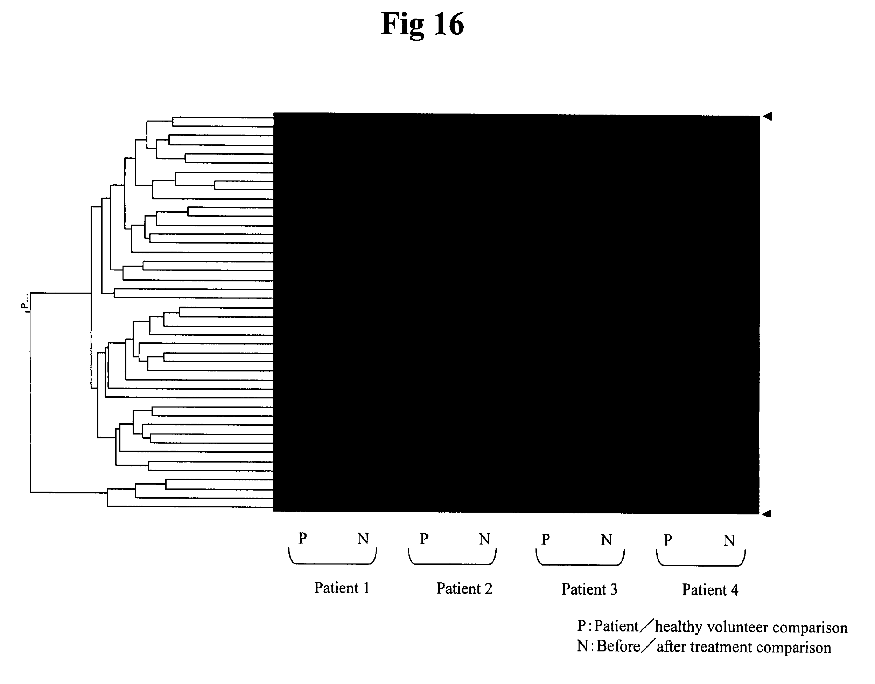

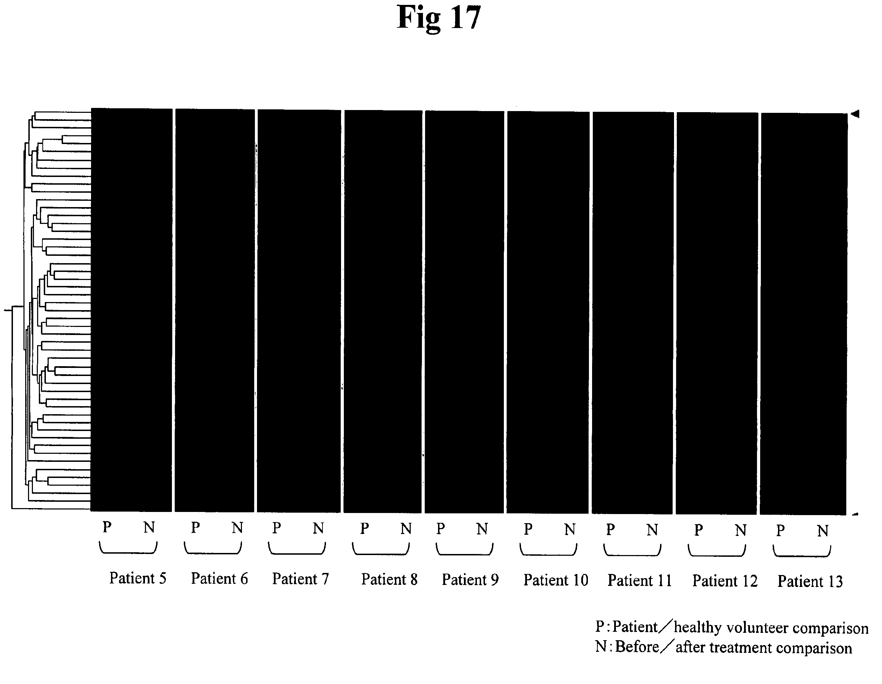

Based on the results attained above, 16 subjects for before/after treatment comparison were divided into the PA group (7 subjects) and the PB group (9 subjects). The data on patient/healthy volunteer comparison and the data on before/after treatment comparison were aligned for each patient in each group, and the data were compared and analyzed. The group of genes with reversed expression patterns between the data on patient/healthy volunteer comparison and the data on before/after treatment comparison was extracted (PA group: Fig. 16 (reversed patterns were clearly observed in 4 individuals), Table 9; PB group: Fig. 17, Table 10). Concerning the PA group, variation in expression levels of CLK1, PSMC6, TAF2F, P2Y5, CASP3, HSPCA, MSH2, SLC38A2, B2M, and AKAP11 were particularly significant among the genes listed in Table 9. Concerning the PB group, variation in expression levels of CCNA2, HGF, GPR24, PTGER3, COX7A2, BDKRB2, UFD1L, HMG1, PSMA4, and ATP6J were particularly significant among the genes listed in Table 10.

**Table 9:**

| Group of genes exhibiting significant differences before and after treatment in PA group | | | |
|---|---|---|---|
| Symbol | Name | Category | GenBank ID |
| CLK1 | Homo sapiens clk1 mRNA; CDC-like kinase 1 | CellCycle | L29222 |
| PSMC6 | Proteasome (prosome, macropain) 26S subunit, ATPase, 6 | ATPase | AF006305 |
| TAF2F | TATA box binding protein (TBP)-associated factor, RNA polymerase II, F, 55kD | polymerase, TF | U18062 |
| P2Y5 | Homo sapiens purinergic receptor P2Y5 mRNA | Signal | AF000546 |
| CASP3 | Human cysteine protease CPP32 isoform alpha mRNA, complete cds | Appoptosis, Signal | U13737 |
| HSPCA | Homo sapiens Hsp89-alpha-delta-N mRNA; Heat shock 90kD protein 1, alpha | hsp | AF028832 |
| MSH2 | Human DNA mismatch repair protein MSH2 | DNArepair | U04045 |
| SLC38A2 | amino acid transporter 2 | - | AF259799 |
| B2M | beta-2-microglobulin | - | AY007153 |
| AKAP11 | A kinase (PRKA) anchor protein 11 (AKAP11); Homo sapiens mRNA for KIAA0629 protein, partial cds | Signal | AB014529 |
| PSMA4 | proteasome (prosome, macropain) subunit, alpha type, 4 | - | BC005361 |
| EEF1A1 | Homo sapiens eukaryotic translation elongation factor 1 alpha 1 (EEF1A1) | glucocorticoids (Cortisol) | NM_001402 |
| MAP2K6 | Human MAP.kinase kinase 6 mRNA, complete cds; MEK6 | Signal . | U39064 |
| BMI1 | Human prot-oncogene (BMI-1) mRNA, complete cds | oncogene | L13689 |
| GABPB1 | Homo sapiens GA-binding protein transcription factor, beta subunit 1 (53kD); nuclear respiratory factor-2 | mitcondria & stress | NM_005254 |
| PTPRC | Human mRNA for T200 leukocyte common antigen (CD45, LC-A). | Signal | Y00062 |
| TNFRSF6 | H.sapiens mRNA for APO-1 cell surface antigen, FAS | Appoptosis, Cytokine, Signal | X63717 |
| FGF2 | Human basic fibroblast growth factor (FGF) mRNA (BFGF; FGFB; FGF2) | GF | M27968 |
| GJA4 | gap junction protein, alpha 4, 37kD (connexin 37) | Gap-junciton | M96789 |
| BCL2 | Human bcl-2 mRNA; apoptosis regulator bcl2 | oncogene, Signal | M14745 |
| SMARCA 3 | SWI/SNF related, matrix associated, actin dependent regulator of chromatin, subfamily a, member 3 | ATPase | Z46606 |
| IFIT1 | Human mRNA for 56-KDa protein induced by interferon | Cytokine | X03557 |
| IFNGR1 | Human interferon-gamma receptor mRNA, complete cds | Cytokine, Signal | J03143 |
| FCER1A | Human mRNA for high affinity IgE receptor alpha-subunit (FcERI); Fc fragment of IgE, high affinity I, receptor for; alpha polypeptide | Signal | X06948 |
| GNG2 | Homo sapiens clone FLB4307 PRO1107 mRNA | Signal | AF130106 |
| E2F3 | Homo sapiens E2F transcription factor 3(E2F3) | TF | Y10479 |
| IL8 | Human beta-thromboglobulin-like protein mRNA, complete cds | Cytokine, Signal | M17017 |
| FRAT1 | Homo sapiens frequently rearranged in advanced T-cell lymphomas (FRAT1) mRNA | Signal | NM_005479 |
| COX17 | Homo sapiens COX17 (yeast) homolog, cytochrome c oxidase assembly protein | mitcondria & stress | NM_005694 |
| GZMA | Human Hanukah factor serine protease (HuHF) mRNA (cytotoxic T-lymphocyte-associated serine esterase 3) | esterase | M18737 |
| CDC10 | hCDC10=CDC10 homolog [human, fetal lung, mRNA, 2314 nt]. | CellCycle | S72008 |
| ADH5 | Human alcohol dehydrogenase class III (ADH5) mRNA | ADH | M29872 |
| API1 | Human inhibitor of apoptosis protein 2 mRNA; Apoptosis inhibitor 1 | Appoptosis, Signal | U45879 |
| PPP3CB | Human calcineurin A2 mRNA; | Signal | M29551 |
| GNG10 | Human G protein gamma-10 subunit mRNA; Guanine nucleotide binding protein 10 | Signal | U31383 |
| MAP3K7 | Homo sapiens mitogen-activated protein kinase kinase kinase 7 (MAP3K7), mRNA, TAK1 | Signal | NM_003188 |
| POLB | polymerase (DNA directed), beta | polymerase | D29013 |
| NR3C1 | Human glucocorticoid receptor alpha mRNA, complete cds | glucocorticoids (Cortisol) | M10901 |
| ITGB1 | Integrin, beta 1 (fibronectin receptor, beta polypeptide, antigen CD29 includes MDF2, MSK12); | Signal | X07979 |
| COX6C | Homo sapiens cytochrome c oxidase subunit VIc (COX6C), nuclear gene encoding mitochondrial protein | mitcondria & stress | NM_004374 |
| HSJ2 | Human heat shock protein, E. coli DnaJ homologue mRNA, complete cds; Heat shock protein, DNAJ-like 2 | hsp | L08069 |
| AHR | Human AH-receptor mRNA, complete cds | Ah receptor | L19872 |
| TAF2G | TATA box binding protein (TBP)-associated factor, RNA polymerase II, G, 32kD | polymerase, TF | U21858 |
| IL1R2 | H.sapiens IL-1R2 mRNA for type II interleukin-1 receptor, (cell line CB23). | Cytokine | X59770 |

**Table 10:**

| Group of genes exhibiting significant differences before and after treatment in PB group | | | |
|---|---|---|---|
| Symbol | Name | Category | GenBank ID |
| CCNA2 | Human mRNA for cyclin A; Cyclin A2 | CellCycle | X51688 |
| HGF | Human hepatocyte growth factor mRNA (HGF); scatter factor (SF); hepatopoeitin A | GF | M60718 |
| GPR24 | G protein-coupled receptor 24 | - | BC001736 |
| PTGER3 | prostaglandin E receptor 3 (subtype EP3) | - | X83860 |
| COX7A2 | Homo sapiens cytochrome c oxidase subunit VIIa polypeptide 2 (liver) (COX7A2), nuclear gene encoding mitochondrial protein | mitcondria & stress | NM_001865 |
| BDKRB2 | Homo sapiens bradykinin receptor B2 | heart stress | NM_000623 |
| UFD1L | Ubiquitin fusion degradation 1-like | - | BC005087 |
| HMG1 | Human mRNA for high mobility group-1 protein (HMG-1). | sulfotransferase | X12597 |
| PSMA4 | proteasome (prosome, macropain) subunit, alpha type, 4 | - | BC005361 |
| ATP6J | ATPase, H+ transporting, lysosomal (vacuolar proton pump), member J | ATPase | AF038954 |
| HSPE1 | Human chaperonin 10 mRNA; Heat shock 10kD protein 1 | hsp | U07550 |
| IL13RA2 | Human interleukin-13 receptor mRNA, complete cds | Cytokine | U70981 |
| COX17 | Homo sapiens COX17 (yeast) homolog, cytochrome c oxidase assembly protein | mitcondria & stress | NM_005694 |
| TSSC1 | Homo sapiens tumor suppressing STF cDNA 1 (TSSC1) mRNA, complete cds | Supressor | AF019952 |
| PSMA7 | proteasome (prosome, macropain) subunit, alpha type, 7 | - | BC004427 |
| ATP5J2 | ATP synthase, H+ transporting, mitochondrial F0 complex, subunit isoform 2 | f, ATPase | AF047436 |
| POLE | polymerase (DNA directed), epsilon | polymerase | L09561 |
| HTR6 | 5-hydroxytryptamine (serotonin) receptor 6 | - | NM_000871 |
| APG-1 | Homo sapiens mRNA for heat shock protein apg-1; Heat shock protein (hsp110 family) | hsp | AB023421 |
| CASP4 | Human cysteine protease (ICErel-II) mRNA, complete cds | Appoptosis | U28014 |
| HSPCA | Homo sapiens Hsp89-alpha-delta-N mRNA; Heat shock 90kD protein 1, hsp alpha | | AF028832 |
| FGF2 | Human basic fibroblast growth factor (FGF) mRNA (BFGF; FGFB; FGF2) | GF | M27968 |
| ADH5 | Human alcohol dehydrogenase class III (ADH5) mRNA | ADH | M29872 |
| PSMA6 | proteasome (prosome, macropain) subunit, alpha type 6 | - | X59417 |
| CCNH | Human cyclin H mRNA, complete cds | CellCycle | U11791 |
| COX7C | Homo sapiens cytochrome c oxidase subunit VIIc | mitcondria & stress | NM_001867 |
| SOD1 | Homo sapiens superoxide dismutase 1, soluble (amyotrophic lateral SOD sclerosis 1 (adult)) (SOD1); Superoxide dismutase 1, soluble (amyotrophic lateral sclerosis 1 (adult)) | | NM_000454 |
| HTR2A | 5-hydroxytryptamine (serotonin) receptor 2A | - | X57830 |
| HSJ2 | Human heat shock protein, E. coli DnaJ homologue mRNA, complete cds; Heat shock protein, DNAJ-like 2 | hsp | L08069 |
| DAP3 | Human ionizing radiation resistance conferring protein mRNA; Death associated protein 3 | Appoptosis | U18321 |
| UCHL3 | Human ubiquitin carboxyl-terminal hydrolase (PGP 9.5, UCH-L3) isozyme L3 mRNA | esterase | M30496 |
| CREBBP | Human CREB-binding protein (CBP) mRNA, complete cds | ATF/CREB | U47741 |
| GSTTLp2 | glutathione-S-transferase like; glutathione transferase omega | - | BC000127 |
| 8 | | | |
| PSMA3 | proteasome (prosome, macropain) subunit, alpha type, 3 | - | BC005265 |
| UBL1 | ubiquitin-like 1 (sentrin) | Gap-junciton | U61397 |
| HSBP1 | Homo sapiens heat shock factor binding protein 1 HSBP1 mRNA; Heat shock factor binding protein 1 | hsp | AF068754 |
| NME2 | Human putative NDP kinase (nm23-H2S) mRNA, complete cds; c-myc purine-binding transcription factor puf | TF | M36981 |
| PRDX4 | peroxiredoxin 4 | - | BC003609 |
| COX4 | Homo sapiens cytochrome c oxidase subunit IV (COX4), nuclear gene encoding mitochondrial protein | mitcondria & stress | NM_001861 |
| TGFBR1 | Human activin receptor-like kinase (ALK-5) mRNA, complete cds | GF, Signal | L11695 |
| PSMB7 | proteasome (prosome, macropain) subunit, beta type, 7 | - | BC000509 |
| COX6C | Homo sapiens cytochrome c oxidase subunit VIc (COX6C), nuclear gene encoding mitochondrial protein | mitcondria & stress | NM_004374 |
| GABRR2 | gamma-aminobutyric acid (GABA) receptor, rho 2 | - | NM_002043 |
| CASP5 | Human cysteine protease (ICErel-III) mRNA, complete cds | Appoptosis | U28015 |
| POLR2H | Human RNA polymerase II subunit (hsRPB8) mRNA; polymerase (RNA) (DNA directed) polypeptide H | II polymerase | U37689 |
| PSMB4 | proteasome (prosome, macropain) subunit, beta type, 4 | - | S71381 |
| PSMB1 | proteasome (prosome, macropain) subunit, beta type, 1 | - | BC000508 |
| HSPD1 | Heat shock 60kD protein 1 (chaperonin) | hsp | M34664 |
| ESD | Homo sapiens esterase D mRNA | esterase | AF112219 |
| WISP3 | Homo sapiens connective tissue growth factor related protein WISP-3 (WISP3) mRNA, complete cds. | Signal | AF100781 |
| ATP5JD | ATP synthase, H+ transporting, mitochondrial F1F0, subunit d | ATPase | AF087135 |

### Industrial Applicability

The method according to the present invention is a useful method for objectively diagnosing depression or evaluating the course of treatment for patients afflicted with depression in clinical settings.

## Claims

1. A method of diagnosing depression, wherein gene expression is analyzed using mRNA of a subject's peripheral blood to evaluate whether or not the subject is afflicted with depression, the type of depression of a subject who had been evaluated as being afflicted with depression is identified, and the conditions of depression are then diagnosed in accordance with the type of depression.

2. The method of diagnosing depression according to claim 1, wherein the expression profiles of the marker gene for depression selected from among the genes listed in Table 1 are employed to evaluate whether or not a subject is afflicted with depression and the expression profiles of the marker gene for classification selected from among the genes listed in Table 2 are employed to identify the type of depression to be type PA or PB.

3. The method of diagnosing depression according to claim 2, wherein the marker gene for depression includes at least ATP2A2, SCYA5, STIP1, EEF1A1, GRB10, CASP6, TSSC1, RAB9, NFATC3, and TPR listed in Table 1 and the marker gene for classification includes at least GNG10, CLK1, P2Y5, IFNGR1, TAF2F, PIM1, MAP2K3, HDGF, INSR, and COX6C listed in Table 2.

4. The method of diagnosing depression according to claim 2, wherein the expression profiles of the marker gene for diagnosing type PA depression selected from among the genes listed in Table 3 are employed to diagnose the conditions of the type PA depression and the expression profiles of the marker gene for diagnosing type PB depression selected from among the genes listed in Table 4 are employed to diagnose the conditions of the type PB depression.

5. The method of diagnosing depression according to claim 4, wherein the marker gene for diagnosing type PA depression includes at least CDC10, GZMA, TNFRSF6, HSPCA, NR3C1, TOPBP1, ARNTL, RAP1A, POLR2B, and ITGB1 listed in Table 3 and the marker gene for diagnosing type PB depression includes at least POU2F2, BCL2L1, DAXX, COX4, CD3G, FCER1G, NME2, CPT1B, HSPE1, and COX7A2 listed in Table 4.

6. The method of diagnosing depression according to claim 1, wherein the course of treating a single subject is evaluated by comparing and analyzing the gene expression profiles of the subject before and after the treatment.

7. The method of diagnosing depression according to claim 1, wherein the gene expression analysis is carried out using DNA-immobilized solid substrates including chips, arrays, membrane filters, and capillaries.

8. The method of diagnosing depression according to claim 1, wherein the expression profiles of the marker gene for depression selected from among the genes listed in Table 7 are employed to evaluate whether or not a subject is afflicted with depression and the expression profiles of the marker gene for classification selected from among the genes listed in Table 8 are employed to identify the type'of depression to be type PA or PB.

9. The method of diagnosing depression according to claim 8, wherein the marker gene for depression includes at least HLA-G, HRH4, PSMB9, ATP2A2, SCYA5, SLC6A4, CASP6, CSF2, HSD3B 1, and RAB9 and the marker gene for classification includes at least HSPE1, PSMA4, ADH5, PSMA6, COX17, HMG1, GPR24, COX6C, FGF2, and COX7C.

10. The method of diagnosing depression according to claim 9, wherein the expression profile of the marker gene for diagnosing type PA depression selected from among the genes listed in Table 9 are employed to diagnose the conditions of the type PA depression and the expression profile of the marker gene for diagnosing type PB depression selected from among the genes listed in Table 10 are employed to diagnose the conditions of the type PB depression.

11. The method of diagnosing depression according to claim 10, wherein the marker gene for diagnosing type PA depression includes at least CLK1, PSMC6, TAF2F, P2Y5, CASP3, HSPCA, MSH2, SLC38A2, B2M, and AKAP11 and the marker gene for diagnosing type PB depression includes at least CCNA2, HGF, GPR24, PTGER3, COX7A2, BDKRB2, UFD1L, HMG1, PSMA4, and ATP6J.

12. A solid substrate for diagnosing depression having immobilized thereon probes each independently specifically hybridize to any one of the genes listed in Tables 1 to 4 or the genes listed in Tables 7 to 10 for detecting the target gene.

13. A solid substrate for diagnosing depression according to claim 12 having immobilized thereon probes each independently specifically hybridize to any one of the genes listed in Tables 1 to 4 for detecting the target gene, wherein the genes at least include ATP2A2, SCYA5, STIP1, EEF1A1, GRB10, CASP6, TSSC1, RAB9, NFATC3, and TPR listed in Table 1, GNG10, CLK1, P2Y5, IFNGR1, TAF2F, PIM1, MAP2K3, HDGF, INSR, and COX6C listed in Table 2, CDC10, GZMA, TNFRSF6, HSPCA, NR3C1, TOPBP1, ARNTL, RAP1A, POLR2B, and ITGB1 listed in Table 3, and POU2F2, BCL2L1, DAXX, COX4, CD3G, FCER1G, NME2, CPT1B, HSPE1, and COX7A2 listed in Table 4.

14. A solid substrate for diagnosing depression according to claim 12 having immobilized thereon probes each independently specifically hybridize to any one of the genes listed in Tables 7 to 10 for detecting the target gene, wherein the genes at least include HLA-G, HRH4, PSMB9, ATP2A2, SCYA5, SLC6A4, CASP6, CSF2, HSD3B1, and RAB9 listed in Table 7, HSPE1, PSMA4, ADH5, PSMA6, COX17, HMG1, GPR24, COX6C, FGF2, and COX7C listed in Table 8, CLK1, PSMC6, TAF2F, P2Y5, CASP3, HSPCA, MSH2, SLC38A2, B2M, and AKAP11 listed in Table 9, and CCNA2, HGF, GPR24, PTGER3, COX7A2, BDKRB2, UFD1L, HMG1, PSMA4, and ATP6J listed in Table 10.

15. A system for diagnosing depression for performing the method of diagnosing depression according to claim 1, which comprises a means for comparing and analyzing the gene expression data of a subject with that of a healthy volunteer and of a patient afflicted with depression, which had been previously obtained, and diagnoses the conditions of depression of the subject in accordance with the type of depression.

16. The system for diagnosing depression according to claim 15, which further comprises a means of comparing and analyzing the gene expression data of a subject, of a healthy volunteer, and of a patient afflicted with depression in combination with the data concerning their age and sex.
